# EUROPEAN PATENT APPLICATION

(11) **EP 4 428 153 A1**
(43) Date of publication of application: **11.09.2024**
(21) Application number: 22889403.6
(22) Date of filing: 04.11.2022
(51) Int. Cl.: C07K 16/28, A61K 39/395, A61P 35/00

(54) **ANTIBODY BINDING TO GPRC5D AND USE THEREOF**

(30) Priority: 05.11.2021 CN 202111305149
(71) Applicant: CHIA TAI TIANQING PHARMACEUTICAL GROUP CO., LTD., Lianyungang, Jiangsu 222062 (CN)
(72) Inventor: LU, Zhenzhen, Nanjing, Jiangsu 211100 (CN); WANG, Yujie, Nanjing, Jiangsu 211100 (CN); PENG, Shuangli, Nanjing, Jiangsu 211100 (CN); ZHANG, Zhengping, Nanjing, Jiangsu 211100 (CN)
(74) Representative: Lavoix
(86) International application number: PCT/CN2022/129803
(87) International publication number: WO 2023/078382

(57) **Abstract**

Provided are an antibody binding to GPRCSD and a use thereof. Specifically provided are an anti-GPRCSD antibody and an antigen-binding fragment thereof, a nucleic acid encoding same, a vector comprising the nucleic acid, a cell comprising the vector, and a pharmaceutical composition comprising same. Further provided is a use thereof in reducing tumors or inhibiting tumor cell growth, treating diseases, etc.

## Description

### TECHNICAL FIELD

The present disclosure relates to an antibody, in particular to an antibody binding to GPRC5D, a method for preparing same, and use thereof.

### BACKGROUND

Multiple myeloma (MM) is a plasmocytic malignancy characterized by the abnormal proliferation of bone marrow-derived plasmocytes, which in turn causes a variety of disease-related symptoms such as osteonecrosis, bone marrow infiltration, renal failure, and immunodeficiency in MM patients. Currently, therapies for multiple myeloma include proteasome inhibitors, immunomodulatory drugs, monoclonal antibodies, and stem cell transplantation, etc. GPRC5D (G protein-coupled receptor class C group 5 member D) is a member of the retinoic acid-inducible orphan G protein-coupled receptor (RAIG) family. It is a 7-transmembrane protein consisting of 345 amino acid residues with its normal physiological function associated with the structure of sclerotin. However, no specific biological function or ligand of GPRC5D has been disclosed at present. Some studies have shown that GPRC5D has an expression threshold of over 50% on malignant bone marrow-derived plasmocytes in 65% of patients with multiple myeloma, and that its expression is independent of BCMA (B-cell maturation antigen). Other studies have shown that the overexpression of GPRC5D correlates with tumor burden and poor prognosis in patients with multiple myeloma. GPRC5D has no or very low expression in normal tissues, except for hair follicle tissues of the skin and bone marrow-derived plasmocytes. GPRC5D may provide a new therapeutic pathway with clinical prospects for patients with multiple myeloma.

As a potential therapeutic target, some antibodies targeting GPRC5D have been developed, which, however, are still limited. There is a need for more available options.

### SUMMARY

The present disclosure provides an antibody binding to GPRC5D, and further provides related nucleic acids encoding the provided antibody, vectors, cells, compositions, preparation methods, and use.

In one aspect, the present disclosure provides an isolated anti-GPRC5D antibody or an antigen-binding fragment thereof, wherein the anti-GPRC5D antibody comprises:
a heavy chain variable region comprising:
   a HCDR1 comprising the amino acid sequence set forth in SEQ ID NO: 1, 9, 17, 25, or 33,
   a HCDR2 comprising the amino acid sequence set forth in SEQ ID NO: 2, 10, 18, 26, or 34, and
   a HCDR3 comprising the amino acid sequence set forth in SEQ ID NO: 3, 11, 19, 27, or 35; and
   a light chain variable region comprising:
a LCDR1 comprising the amino acid sequence set forth in SEQ ID NO: 4, 12, 20, 28, or 36,
a LCDR2 comprising the amino acid sequence set forth in SEQ ID NO: 5, 13, 21, 29, or 37, and
a LCDR3 comprising the amino acid sequence set forth in SEQ ID NO: 6, 14, 22, 30, or 38.

In one embodiment of the present disclosure, the anti-GPRC5D antibody comprises:
(i) a heavy chain variable region comprising:
   a HCDR1 comprising the amino acid sequence set forth in SEQ ID NO: 1,
   a HCDR2 comprising the amino acid sequence set forth in SEQ ID NO: 2, and
   a HCDR3 comprising the amino acid sequence set forth in SEQ ID NO: 3; and
   a light chain variable region comprising:
      a LCDR1 comprising the amino acid sequence set forth in SEQ ID NO: 4,
      a LCDR2 comprising the amino acid sequence set forth in SEQ ID NO: 5, and
      a LCDR3 comprising the amino acid sequence set forth in SEQ ID NO: 6;
(ii) a heavy chain variable region comprising:
   a HCDR1 comprising the amino acid sequence set forth in SEQ ID NO: 9,
   a HCDR2 comprising the amino acid sequence set forth in SEQ ID NO: 10, and
   a HCDR3 comprising the amino acid sequence set forth in SEQ ID NO: 11; and
   a light chain variable region comprising:
      a LCDR1 comprising the amino acid sequence set forth in SEQ ID NO: 12,
      a LCDR2 comprising the amino acid sequence set forth in SEQ ID NO: 13, and
      a LCDR3 comprising the amino acid sequence set forth in SEQ ID NO: 14;
(iii) a heavy chain variable region comprising:
   a HCDR1 comprising the amino acid sequence set forth in SEQ ID NO: 17,
   a HCDR2 comprising the amino acid sequence set forth in SEQ ID NO: 18, and
   a HCDR3 comprising the amino acid sequence set forth in SEQ ID NO: 19; and
   a light chain variable region comprising:
      a LCDR1 comprising the amino acid sequence set forth in SEQ ID NO: 20,
      a LCDR2 comprising the amino acid sequence set forth in SEQ ID NO: 21, and
      a LCDR3 comprising the amino acid sequence set forth in SEQ ID NO: 22;
(iv) a heavy chain variable region comprising:
   a HCDR1 comprising the amino acid sequence set forth in SEQ ID NO: 25,
   a HCDR2 comprising the amino acid sequence set forth in SEQ ID NO: 26, and
   a HCDR3 comprising the amino acid sequence set forth in SEQ ID NO: 27; and
   a light chain variable region comprising:
      a LCDR1 comprising the amino acid sequence set forth in SEQ ID NO: 28,
      a LCDR2 comprising the amino acid sequence set forth in SEQ ID NO: 29, and
      a LCDR3 comprising the amino acid sequence set forth in SEQ ID NO: 30; or
(v) a heavy chain variable region comprising:
   a HCDR1 comprising the amino acid sequence set forth in SEQ ID NO: 33,
   a HCDR2 comprising the amino acid sequence set forth in SEQ ID NO: 34, and
   a HCDR3 comprising the amino acid sequence set forth in SEQ ID NO: 35; and
   a light chain variable region comprising:
      a LCDR1 comprising the amino acid sequence set forth in SEQ ID NO: 36,
      a LCDR2 comprising the amino acid sequence set forth in SEQ ID NO: 37, and
      a LCDR3 comprising the amino acid sequence set forth in SEQ ID NO: 38.

In one aspect, the present disclosure provides an isolated anti-GPRC5D antibody or an antigen-binding fragment thereof, wherein the anti-GPRC5D antibody comprises a heavy chain variable region comprising the HCDR1, the HCDR2, and the HCDR3 of the variable region sequence set forth in SEQ ID NO: 7, 15, 23, 31, or 39, and a light chain variable region comprising the LCDR1, the LCDR2, and the LCDR3 of the variable region sequence set forth in SEQ ID NO: 8, 16, 24, 32, or 40.

In one aspect, the present disclosure provides a fusion protein comprising the anti-GPRC5D antibody or the antigen-binding fragment thereof described herein.

In one aspect, the present disclosure provides a pharmaceutical composition comprising the anti-GPRC5D antibody or the antigen-binding fragment thereof or the fusion protein described herein and further comprising a pharmaceutically acceptable carrier.

In one aspect, the present disclosure provides an isolated nucleic acid encoding the anti-GPRC5D antibody or the antigen-binding fragment thereof described herein.

In one aspect, the present disclosure provides a vector comprising the nucleic acid described herein.

In one aspect, the present disclosure provides a host cell comprising the vector described herein or integrated in the genome with the nucleic acid described herein.

In one aspect, the present disclosure provides a method for preparing the anti-GPRC5D antibody or the antigen-binding fragment thereof described herein, comprising: culturing the host cell and isolating the anti-GPRC5D antibody or the antigen-binding fragment thereof from the host cell or the host cell culture.

In one aspect, the present disclosure provides use of the anti-GPRC5D antibody or the antigen-binding fragment thereof or the fusion protein for preparing a medicament for treating a disease, wherein preferably, the disease is a cancer or an autoimmune disease.

In one aspect, the present disclosure provides a method for reducing a tumor or inhibiting tumor cell growth in a subject, comprising administering to the subject a therapeutically effective amount of the anti-GPRC5D antibody or the antigen-binding fragment thereof, the fusion protein, or the pharmaceutical composition described herein.

In one aspect, the present disclosure provides a method for treating a disease in a subject, comprising administering to the subject a therapeutically effective amount of the anti-GPRC5D antibody or the antigen-binding fragment thereof, the fusion protein, or the pharmaceutical composition described herein, wherein preferably, the disease is a cancer or an autoimmune disease.

In one aspect, the present disclosure provides a kit comprising the anti-GPRC5D antibody or the antigen-binding fragment thereof, the immunoconjugate, or the pharmaceutical composition described herein.

### BRIEF DESCRIPTION OF THE DRAWINGS

FIG. 1 shows the OD₄₅₀ values of some mouse anti-hGPRC5D antibodies with CHO-K1-hGPRC5D^{hi}, CHO-K1-cynoGPRC5D (CHO-K1-monkey GPRC5D), and CHO-K1-murineGPRC5D (CHO-K1-mouse GPRC5D) cells as measured by ELISA;
FIG. 2 shows the MFI and EC₅₀ values of the binding of chimeric antibodies to cells with high expression of hGPRC5D as measured by FACS;
FIG. 3 shows the MFI values of the binding of chimeric antibodies to cells with low expression of hGPRC5D as measured by FACS;
FIG. 4A shows the MFI values of the binding of anti-GPRC5D antibodies to H929 cells as measured by FACS;
FIG. 4B shows the MFI values of the binding of anti-GPRC5D antibodies to MM1S cells as measured by FACS;
FIG. 5A shows the MFI values of the binding of anti-GPRC5D antibodies to CHO-K1-cynoGPRC5D cells as measured by FACS;
FIG. 5B shows the MFI values of the binding of anti-GPRC5D antibodies to CHO-K1-murineGPRC5D cells as measured by FACS;
FIG. 6A shows the MFI values of the binding of anti-GPRC5D antibodies to CHO-K1-GPRC5A cells as measured by FACS;
FIG. 6B shows the MFI values of the binding of anti-GPRC5D antibodies to CHO-K1-GPRC5B cells as measured by FACS;
FIG. 6C shows the MFI values of the binding of anti-GPRC5D antibodies to CHO-K1-GPRC5C cells as measured by FACS;
FIG. 7 shows the MFI values of the binding of anti-GPRC5D antibodies to CHO-K1 cells as measured by FACS;
FIG. 8 shows the internalizing activity assay for anti-GPRC5D antibodies.

### DETAILED DESCRIPTION

The exemplary embodiments of the present disclosure have been described herein; however, it will be appreciated by those skilled in the art that the protection scope of the present disclosure is not limited thereto, and that various modifications, alterations, or changes can be made based on the spirit and conception of the present disclosure. The content with these modifications, alterations or changes shall still fall within the scope of the present disclosure.

### Terminology

The term "antibody" is used in its broadest sense and encompasses a variety of antibody structures, including natural and artificial antibodies of various structures, including, but not limited to, monoclonal antibodies, monospecific antibodies, multispecific antibodies (e.g., bispecific antibodies, trispecific antibodies, etc.), single-chain antibodies, and the like, so long as they exhibit the desired antigen-binding activity. The antigen-binding fragment refers to a molecule, other than an intact antibody, comprising a portion of the intact antibody that binds to the antigen to which the intact antibody binds. Examples of the antigen-binding fragment include an Fab fragment, an Fab' fragment, an F(ab)' fragment, an Fv fragment, an isolated CDR region, a single-chain Fv molecule (scFv), and other antibody fragments known in the art.

The term "isotype" refers to the class of antibodies encoded by the heavy chain constant region gene. In one embodiment, the anti-GPRC5D antibody disclosed herein is of the IgG1 or IgG4 isotype. The anti-GPRC5D antibody and the antigen-binding fragment thereof of the present disclosure may be derived from any species, including but not limited to mouse, rat, rabbit, primates, llama, and human. The anti-GPRC5D antibody may be a chimeric antibody, a humanized antibody, or an intact human antibody. In one specific embodiment, the anti-GPRC5D antibody provided herein is a humanized antibody. In one specific embodiment, the anti-GPRC5D antibody provided herein is a chimeric antibody.

The term "chimeric antibody" refers to an antibody having at least a portion of the heavy chain variable region and at least a portion of the light chain variable region derived from one species, and at least a portion of the constant region derived from another species. For example, in some embodiments, the chimeric antibody may comprise a murine variable region and a human constant region.

The term "humanized antibody" refers to an antibody comprising complementarity determining regions (CDRs) derived from a non-human antibody, and framework and constant regions derived from a human antibody. For example, the humanized antibody that binds to GPRC5D provided herein may comprise CDRs derived from one or more murine antibodies and human framework and constant regions.

The term "monoclonal antibody" ("mAb") refers to an antibody molecule of a single-molecule composition. A monoclonal antibody composition exhibits a sole binding specificity and affinity for a particular epitope or, in the case of bispecific monoclonal antibody, a dual binding specificity for two different epitopes. mAb is an example of the isolated antibody. mAbs can be produced by hybridoma techniques, recombinant techniques, transgenic techniques, or other techniques known to those skilled in the art.

The term "variable domain" or "variable region" refers to a domain of an antibody that is involved in the binding of the antibody to an antigen. For example, a natural four-chain antibody (e.g., derived from human, murine, and the like) has a heavy chain variable region (VH) and a light chain variable region (VL), and a heavy-chain antibody derived from an animal such as camelid species or sharks has a single variable domain. In most cases, each variable domain of a natural antibody consists substantially of four "framework regions" and three "complementarity determining regions". The four framework regions are referred to as framework region 1 (FR1), framework region 2 (FR2), framework region 3 (FR3), and framework region 4 (FR4), respectively; the framework regions are separated by what are referred to in the art and hereinafter as complementarity determining regions (CDRs). Thus, the general structure of a variable domain can be represented as follows: FR1-CDR1-FR2-CDR2-FR3-CDR3-FR4. In one specific example, the general structure of a heavy chain variable region can be represented as follows: FR1-HCDR1-FR2-HCDR2-FR3-HCDR3-FR4. In one specific example, the general structure of a light chain variable region can be represented as follows: FR1-LCDR1-FR2-LCDR2-FR3-LCDR3-FR4.

"CDR" (complementarity determining region) is also referred to as "hypervariable region (HVR)". For example, a natural four-chain antibody typically comprises six CDRs, among which three are in the heavy chain variable region, i.e., heavy chain CDR1 (HCDR1), heavy chain CDR2 (HCDR2), and heavy chain CDR3 (HCDR3), and three are in the light chain variable region, i.e., light chain CDR1 (LCDR1), light chain CDR2 (LCDR2), and light chain CDR3 (LCDR3). A heavy-chain antibody or a single variable domain typically has three CDRs (CDR1, CDR2, and CDR3).

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. The "Contact" defines CDRs based on the analysis of the crystal structure of available complexes. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined with a particular CDR sequence defined herein, the scope of the antibody also encompasses antibodies defined by CDR sequences that are converted to any other definitions (e.g., a combination of one or more of the IMGT, Chothia, AbM definitions, etc.). Although the CDRs claimed in the present disclosure are based on the sequences shown in Table S1 and Table S2, the corresponding amino acid sequences according to other CDR definition rules shall also fall within the protection scope of the present disclosure. For example, under another definition, the amino acid sequence of the heavy chain CDR1 of chimeric antibody Chi-131 may be NYVMH (SEQ ID NO: 71).

The term "framework region" (FR) refers to amino acid residues of variable domains other than the CDR residues defined herein.

The term "isolated" means that a target compound (e.g., an antibody or nucleic acid) has been isolated from its natural environment.

As used herein, the term "EC₅₀" refers to a concentration of an antibody that induces 50% of the maximal effective response. As used herein, the term "IC₅₀" refers to a concentration of an antibody that induces 50% of the maximal inhibitory response. Both EC₅₀ and IC₅₀ may be measured by ELISA or FACS assay or any other method known in the art.

The term "K_{D}" as used herein refers to the equilibrium dissociation constant, expressed in molar concentration (M). The K_{D} value of an antibody can be determined using methods well known in the art. One preferred method for determining the K_{D} of an antibody is performed by using surface plasmon resonance, more preferably a biosensor system, such as the Biacore system.

As used herein, the term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present disclosure is a human. The terms "patient" and "subject" are used interchangeably unless otherwise indicated.

As used herein, the "cancer" refers to a physiological condition in mammals that is typically characterized by unregulated cell growth. Uncontrolled cell division and growth may lead to the formation of malignant tumors that invade adjacent tissues, and may also metastasize to distant parts of the body through the lymphatic system or blood flow. The "cancer" or "cancer tissue" may include a tumor.

The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving symptoms, reducing any direct or indirect pathological outcomes of diseases, preventing metastasis, slowing disease progression rates, improving or alleviating disease conditions, and regressing or improving prognosis.

As used herein, the term "therapeutically effective amount" refers to an amount of a compound, a composition, or a pharmaceutical combination necessary to provide a therapeutic benefit for a subject.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The "pharmaceutical composition" refers to a composition comprising an active ingredient and a pharmaceutically acceptable carrier.

The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without any conservative replacements as part of the sequence identity. The alignment of amino acid sequences for identity can be performed in a variety of ways within the skill in the art, e.g., BLAST, BLAST-2, ALIGN, or Megalign (DNASTAR) software. Those skilled in the art can determine suitable parameters for aligning sequences, including any algorithms required to obtain maximum alignment for the full length of sequences being compared.

The terms "Xn" and "Xaa" are equivalent and refer to an unspecified amino acid whose scope is specified by the subsequent definitions in the relevant description.

The term "include", "contain", or "comprise" and variants thereof should be interpreted as "including, but not limited to", which means that other elements, components, and procedures that are not specified are encompassed in addition to the elements, components, and procedures that are listed.

Unless otherwise specified clearly herein, singular terms encompass plural terms, and vice versa.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art. Various aspects of the present disclosure will be described in further detail in the following sections.

### I. Anti-GPRCSD antibody and antigen-binding fragment thereof

Herein, the terms "human GPRC5D" and "hGPRC5D" are used interchangeably herein; the terms "antibody binding to GPRC5D" and "anti-GPRC5D antibody" are used interchangeably.

The present disclosure provides a novel antibody that binds to GPRC5D. The anti-GPRC5D antibody exhibits a plurality of other desirable properties for therapeutic or/and diagnostic applications. For example, in some embodiments, the anti-GPRC5D antibody has good specificity for GPRC5D. In some embodiments, the anti-GPRC5D antibody can bind to monkey GPRC5D or/and mouse GPRC5D, thereby facilitating pharmacological or/and safety assessments during antibody development. In some embodiments, the anti-GPRC5D antibody provided herein exhibits better antigen-binding properties, e.g., better binding ability to cells with low GPRC5D expression, as compared to some known antibodies. In some embodiments, the anti-GPRC5D antibody has good killing properties against tumor cells, e.g., multiple myeloma cells.

In one aspect, the present disclosure provides an isolated anti-GPRC5D antibody or an antigen-binding fragment thereof, wherein the anti-GPRC5D antibody comprises:
a heavy chain variable region comprising:
   a HCDR1 comprising the amino acid sequence set forth in SEQ ID NO: 1, 9, 17, 25, or 33,
   a HCDR2 comprising the amino acid sequence set forth in SEQ ID NO: 2, 10, 18, 26, or 34, and
   a HCDR3 comprising the amino acid sequence set forth in SEQ ID NO: 3, 11, 19, 27, or 35; and
   a light chain variable region comprising:
a LCDR1 comprising the amino acid sequence set forth in SEQ ID NO: 4, 12, 20, 28, or 36,
a LCDR2 comprising the amino acid sequence set forth in SEQ ID NO: 5, 13, 21, 29, or 37, and
a LCDR3 comprising the amino acid sequence set forth in SEQ ID NO: 6, 14, 22, 30, or 38.

In some embodiments, the anti-GPRC5D antibody comprises:
a heavy chain variable region comprising: a HCDR1 comprising the amino acid sequence set forth in SEQ ID NO: 1, a HCDR2 comprising the amino acid sequence set forth in SEQ ID NO: 2, and a HCDR3 comprising the amino acid sequence set forth in SEQ ID NO: 3; and
a light chain variable region comprising: a LCDR1 comprising the amino acid sequence set forth in SEQ ID NO: 4, a LCDR2 comprising the amino acid sequence set forth in SEQ ID NO: 5, and a LCDR3 comprising the amino acid sequence set forth in SEQ ID NO: 6.

In some embodiments, the anti-GPRC5D antibody comprises:
a heavy chain variable region comprising: a HCDR1 comprising the amino acid sequence set forth in SEQ ID NO: 9, a HCDR2 comprising the amino acid sequence set forth in SEQ ID NO: 10, and a HCDR3 comprising the amino acid sequence set forth in SEQ ID NO: 11; and
a light chain variable region comprising: a LCDR1 comprising the amino acid sequence set forth in SEQ ID NO: 12, a LCDR2 comprising the amino acid sequence set forth in SEQ ID NO: 13, and a LCDR3 comprising the amino acid sequence set forth in SEQ ID NO: 14.

In some embodiments, the anti-GPRC5D antibody comprises:
a heavy chain variable region comprising: a HCDR1 comprising the amino acid sequence set forth in SEQ ID NO: 17, a HCDR2 comprising the amino acid sequence set forth in SEQ ID NO: 18, and a HCDR3 comprising the amino acid sequence set forth in SEQ ID NO: 19; and
a light chain variable region comprising: a LCDR1 comprising the amino acid sequence set forth in SEQ ID NO: 20, a LCDR2 comprising the amino acid sequence set forth in SEQ ID NO: 21, and a LCDR3 comprising the amino acid sequence set forth in SEQ ID NO: 22.

In some embodiments, the anti-GPRC5D antibody comprises:
a heavy chain variable region comprising: a HCDR1 comprising the amino acid sequence set forth in SEQ ID NO: 25, a HCDR2 comprising the amino acid sequence set forth in SEQ ID NO: 26, and a HCDR3 comprising the amino acid sequence set forth in SEQ ID NO: 27; and
a light chain variable region comprising: a LCDR1 comprising the amino acid sequence set forth in SEQ ID NO: 28, a LCDR2 comprising the amino acid sequence set forth in SEQ ID NO: 29, and a LCDR3 comprising the amino acid sequence set forth in SEQ ID NO: 30.

In some embodiments, the anti-GPRC5D antibody comprises:
a heavy chain variable region comprising: a HCDR1 comprising the amino acid sequence set forth in SEQ ID NO: 33, a HCDR2 comprising the amino acid sequence set forth in SEQ ID NO: 34, and a HCDR3 comprising the amino acid sequence set forth in SEQ ID NO: 35; and
a light chain variable region comprising: a LCDR1 comprising the amino acid sequence set forth in SEQ ID NO: 36, a LCDR2 comprising the amino acid sequence set forth in SEQ ID NO: 37, and a LCDR3 comprising the amino acid sequence set forth in SEQ ID NO: 38.

In some embodiments, an isolated anti-GPRC5D antibody or an antigen-binding fragment thereof is provided, wherein the anti-GPRC5D antibody comprises a heavy chain variable region comprising the HCDR1, the HCDR2, and the HCDR3 of the variable region sequence set forth in SEQ ID NO: 7, 15, 23, 31, or 39, and a light chain variable region comprising the LCDR1, the LCDR2, and the LCDR3 of the variable region sequence set forth in SEQ ID NO: 8, 16, 24, 32, or 40. In some specific embodiments, the heavy chain variable region comprises the HCDR1, the HCDR2, and the HCDR3 of the variable region sequence set forth in SEQ ID NO: 7, and the light chain variable region comprises the LCDR1, the LCDR2, and the LCDR3 of the variable region sequence set forth in SEQ ID NO: 8. In some specific embodiments, the heavy chain variable region comprises the HCDR1, the HCDR2, and the HCDR3 of the variable region sequence set forth in SEQ ID NO: 15, and the light chain variable region comprises the LCDR1, the LCDR2, and the LCDR3 of the variable region sequence set forth in SEQ ID NO: 16. In some specific embodiments, the heavy chain variable region comprises the HCDR1, the HCDR2, and the HCDR3 of the variable region sequence set forth in SEQ ID NO: 23, and the light chain variable region comprises the LCDR1, the LCDR2, and the LCDR3 of the variable region sequence set forth in SEQ ID NO: 24. In some specific embodiments, the heavy chain variable region comprises the HCDR1, the HCDR2, and the HCDR3 of the variable region sequence set forth in SEQ ID NO: 31, and the light chain variable region comprises the LCDR1, the LCDR2, and the LCDR3 of the variable region sequence set forth in SEQ ID NO: 32. In some specific embodiments, the heavy chain variable region comprises the HCDR1, the HCDR2, and the HCDR3 of the variable region sequence set forth in SEQ ID NO: 39, and the light chain variable region comprises the LCDR1, the LCDR2, and the LCDR3 of the variable region sequence set forth in SEQ ID NO: 40. In some embodiments, in such specific embodiments, the CDR regions of antibodies may be under the kabat, Chothia, IMGT, or other definitions.

In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 7, 15, 23, 31, or 39. In some specific embodiments, the heavy chain variable region comprises the amino acid sequence set forth in SEQ ID NO: 7. 15, 23, 31, or 39.

In some embodiments, the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 8, 16, 24, 32, or 40. In some specific embodiments, the light chain variable region comprises the amino acid sequence set forth in SEQ ID NO: 8. 16, 24, 32, or 40.

In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 7, 15, 23, 31, or 39, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 8, 16, 24, 32, or 40. In some specific embodiments, the heavy chain variable region comprises the amino acid sequence set forth in SEQ ID NO: 7, 15, 23, 31, or 39, and the light chain variable region comprises the amino acid sequence set forth in SEQ ID NO: 8, 16, 24, 32, or 40.

In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 7, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 8. Further, in some of such embodiments, the anti-GPRC5D antibody further comprises: a HCDR1 comprising the amino acid sequence set forth in SEQ ID NO: 1, a HCDR2 comprising the amino acid sequence set forth in SEQ ID NO: 2, a HCDR3 comprising the amino acid sequence set forth in SEQ ID NO: 3, a LCDR1 comprising the amino acid sequence set forth in SEQ ID NO: 4, a LCDR2 comprising the amino acid sequence set forth in SEQ ID NO: 5, and a LCDR3 comprising the amino acid sequence set forth in SEQ ID NO: 6.

In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 15, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 16. Further, in some of such embodiments, the anti-GPRC5D antibody further comprises: a HCDR1 comprising the amino acid sequence set forth in SEQ ID NO: 9, a HCDR2 comprising the amino acid sequence set forth in SEQ ID NO: 10, a HCDR3 comprising the amino acid sequence set forth in SEQ ID NO: 11, a LCDR1 comprising the amino acid sequence set forth in SEQ ID NO: 12, a LCDR2 comprising the amino acid sequence set forth in SEQ ID NO: 13, and a LCDR3 comprising the amino acid sequence set forth in SEQ ID NO: 14.

In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 23, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 24. Further, in some of such embodiments, the anti-GPRC5D antibody further comprises: a HCDR1 comprising the amino acid sequence set forth in SEQ ID NO: 17, a HCDR2 comprising the amino acid sequence set forth in SEQ ID NO: 18, a HCDR3 comprising the amino acid sequence set forth in SEQ ID NO: 19, a LCDR1 comprising the amino acid sequence set forth in SEQ ID NO: 20, a LCDR2 comprising the amino acid sequence set forth in SEQ ID NO: 21, and a LCDR3 comprising the amino acid sequence set forth in SEQ ID NO: 22. In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 31, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 32. Further, in some of such embodiments, the anti-GPRC5D antibody further comprises: a HCDR1 comprising the amino acid sequence set forth in SEQ ID NO: 25, a HCDR2 comprising the amino acid sequence set forth in SEQ ID NO: 26, a HCDR3 comprising the amino acid sequence set forth in SEQ ID NO: 27, a LCDR1 comprising the amino acid sequence set forth in SEQ ID NO: 28, a LCDR2 comprising the amino acid sequence set forth in SEQ ID NO: 29, and a LCDR3 comprising the amino acid sequence set forth in SEQ ID NO: 30. In some embodiments, the heavy chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 39, and the light chain variable region comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 40. Further, in some of such embodiments, the anti-GPRC5D antibody further comprises: a HCDR1 comprising the amino acid sequence set forth in SEQ ID NO: 33, a HCDR2 comprising the amino acid sequence set forth in SEQ ID NO: 34, a HCDR3 comprising the amino acid sequence set forth in SEQ ID NO: 35, a LCDR1 comprising the amino acid sequence set forth in SEQ ID NO: 36, a LCDR2 comprising the amino acid sequence set forth in SEQ ID NO: 37, and a LCDR3 comprising the amino acid sequence set forth in SEQ ID NO: 38. In some specific embodiments, the heavy chain variable region comprises the amino acid sequence set forth in SEQ ID NO: 7, and the light chain variable region comprises the amino acid sequence set forth in SEQ ID NO: 8.

In some specific embodiments, the heavy chain variable region comprises the amino acid sequence set forth in SEQ ID NO: 15, and the light chain variable region comprises the amino acid sequence set forth in SEQ ID NO: 16.

In some specific embodiments, the heavy chain variable region comprises the amino acid sequence set forth in SEQ ID NO: 23, and the light chain variable region comprises the amino acid sequence set forth in SEQ ID NO: 24.

In some specific embodiments, the heavy chain variable region comprises the amino acid sequence set forth in SEQ ID NO: 31, and the light chain variable region comprises the amino acid sequence set forth in SEQ ID NO: 32.

In some specific embodiments, the heavy chain variable region comprises the amino acid sequence set forth in SEQ ID NO: 39, and the light chain variable region comprises the amino acid sequence set forth in SEQ ID NO: 40.

In some embodiments, the anti-GPRC5D antibody provided herein comprises a heavy chain and a light chain that comprise a constant region in addition to the corresponding heavy chain variable region and light chain variable region described herein. From N-terminus to C-terminus, the heavy chain consists of a heavy chain variable region and a heavy chain constant region, and the light chain consists of a light chain variable region and a light chain constant region.

In some embodiments, the light chain constant region of the anti-GPRC5D antibody is a human κ chain constant region. In some embodiments, the light chain constant region of the anti-GPRC5D antibody is a human λ chain constant region. The heavy chain constant region of the anti-GPRC5D antibody may be derived from a constant region of any type, e.g., IgG, IgM, IgD, IgA, and IgE, and of any isotype, e.g., IgG1, IgG2, IgG3, and IgG4. In some embodiments, the anti-GPRC5D antibody is of the IgG1 isotype. In some embodiments, the anti-GPRC5D antibody is of the IgG4 isotype.

In some embodiments, the heavy chain constant region has an amino acid sequence set forth in SEQ ID NO: 69, and the light chain constant region has an amino acid sequence set forth in SEQ ID NO: 70.

In some embodiments, the heavy chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 49, and the light chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 51. In one specific embodiment, the anti-GPRC5D antibody comprises a heavy chain having the amino acid sequence set forth in SEQ ID NO: 49 and a light chain having the amino acid sequence set forth in SEQ ID NO: 51. In some other embodiments, the heavy chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 53, and the light chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 55. In one specific embodiment, the anti-GPRC5D antibody comprises a heavy chain having the amino acid sequence set forth in SEQ ID NO: 53 and a light chain having the amino acid sequence set forth in SEQ ID NO: 55. In some other embodiments, the heavy chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 57, and the light chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 59. In one specific embodiment, the anti-GPRCSD antibody comprises a heavy chain having the amino acid sequence set forth in SEQ ID NO: 57 and a light chain having the amino acid sequence set forth in SEQ ID NO: 59. In some other embodiments, the heavy chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 61, and the light chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 63. In one specific embodiment, the anti-GPRCSD antibody comprises a heavy chain having the amino acid sequence set forth in SEQ ID NO: 61 and a light chain having the amino acid sequence set forth in SEQ ID NO: 63. In some other embodiments, the heavy chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 65, and the light chain comprises an amino acid sequence having at least 80%, 85%, 86%, 87%, 88%, 89%, 90%, 91%, 92%, 93%, 94%, 95%, 96%, 97%, 98%, 99%, or 100% identity to the sequence set forth in SEQ ID NO: 67. In one specific embodiment, the anti-GPRCSD antibody comprises a heavy chain having the amino acid sequence set forth in SEQ ID NO: 65 and a light chain having the amino acid sequence set forth in SEQ ID NO: 67.

In some embodiments, the anti-GPRCSD antibody cross-reacts with a monkey GPRCSD antigen or/and a mouse GPRCSD antigen.

In some embodiments, the anti-GPRCSD antibody or the antigen-binding fragment thereof binds to a human GPRCSD antigen and optionally, further features any one or more of the following properties:
(1) binding to a monkey GPRCSD antigen;
(2) binding to a mouse GPRCSD antigen; or
(3) no binding to one or more of GPRCSA, GPRCSB, and GPRCSC.

In some embodiments, the anti-GPRCSD antibody or the antigen-binding fragment thereof binds to a human GPRCSD antigen, binds to a monkey GPRCSD antigen or/and a mouse GPRCSD antigen, and furthermore, does not bind to GPRCSA, GPRCSB, and GPRCSC.

In some embodiments, the anti-GPRCSD antibody is a monoclonal antibody.

In some embodiments, the anti-GPRCSD antibody is a monospecific antibody.

In some embodiments, the anti-GPRCSD antibody is a multispecific antibody, e.g., a bispecific antibody or trispecific antibody.

In another aspect, the present disclosure provides an anti-GPRC5D antibody or an antigen-binding fragment thereof that binds to the same epitope on GPRCSD as any of the exemplary antibodies provided herein, e.g., binding to the same epitope as Chi-131, e.g., binding to the same epitope as Chi-169, e.g., binding to the same epitope as Chi-89, and the like. In another aspect, the present disclosure provides an anti-GPRCSD antibody or an antigen-binding fragment thereof that competes with any of the exemplary antibodies provided herein, e.g., competing with Chi-131 for binding to GPRCSD, e.g., competing with Chi-131 for binding to GPRCSD, e.g., competing with Chi-169 for binding to GPRCSD. The binding to GPRCSD can be measured by ELISA, flow cytometry, or surface plasmon resonance (SPR) assay, or any other method known in the art.

The present disclosure further provides some exemplary monoclonal antibodies that bind to GPRCSD, including mouse antibodies mu-89, mu-105, mu-128, mu-131, mu-164, mu-169, and mu-180, as well as constructed chimeric antibodies Chi-89, Chi-105, Chi-128, Chi-131, Chi-164, Chi-169, and Chi-180. The amino acid sequences of HCDRs (HCDR1, HCDR2, and HCDR3) of some exemplary anti-GPRCSD antibodies provided herein are provided in Table S1 below, the amino acid sequences of LCDRs (LCDR1, LCDR2, and LCDR3) are provided in Table S2 below, and the amino acid sequences of the variable regions of some antibodies are provided in Table S3 below.

**Table S1: Heavy chain CDR sequences**

| Name of antibody | Heavy chain CDR1 | Heavy chain CDR2 | Heavy chain CDR3 |
|---|---|---|---|
| Chi-131 | GYTFTNYVMH (SEQ ID NO: 1) | YFNPYNDGTNYNEKFKG (SEQ ID NO: 2) | GGVRRYFDV (SEQ ID NO: 3) |
| Chi-169 | GFTFSNYGMS (SEQ ID NO: 9) | SISSGGGRIYYPDNVKG (SEQ ID NO: 10) | HAMDN (SEQ ID NO: 11) |
| Chi-89 | GFTFSSYGMS (SEQ ID NO: 17) | TISSGGSYTYYPDSVKG (SEQ ID NO: 18) | QALRYHMDS (SEQ ID NO: 19) |
| Chi-105 | GYTFTDYYMN (SEQ ID NO: 25) | YIYPNNGGTGYNQRFKG (SEQ ID NO: 26) | WGGTRLGYYAMDY (SEQ ID NO: 27) |
| Chi-128 | GFTFSSFGMH (SEQ ID NO: 33) | YISRGSSTIYYADTVKG (SEQ ID NO: 34) | SGYGSSSYYFDY (SEQ ID NO: 35) |

**Table S2: Light chain CDR sequences**

| Name | Light chain CDR1 | Light chain CDR2 | Light chain CDR3 |
|---|---|---|---|
| Chi-131 | RASQDIGSNLN (SEQ ID NO: 4) | ATFSLDS (SEQ ID NO: 5) | QQYANFPPT (SEQ ID NO: 6) |
| Chi-169 | SASSSVSNMY (SEQ ID NO: 12) | DTSKLAS (SEQ ID NO: 13) | QQWSSNPLT (SEQ ID NO: 14) |
| Chi-89 | KASQNVGTNVA (SEQ ID NO: 20) | SASYRYS (SEQ ID NO: 21) | QQYYSYPWT (SEQ ID NO: 22) |
| Chi-105 | KASQNVGTNVV (SEQ ID NO: 28) | WASTRHT (SEQ ID NO: 29) | QQYSRYPYT (SEQ ID NO: 30) |
| Chi-128 | RASSSIRSSYLH (SEQ ID NO: 36) | STSNLAS (SEQ ID NO: 37) | QQFSGYPLT (SEQ ID NO: 38) |

**Table S3: Variable region sequences of some antibodies**

| Name | Region | Amino acid sequence | SEQ ID NO: |
|---|---|---|---|
| Chi-131 | VH | EVQLQQSGPELVKPGASVKMSCKASGYTFTNYVMHWVKQ KPGQGLEWIGYFNPYNDGTNYNEKFKGKATLTSDKSSNTA YMELSSLTSEDSAVYYCARGGVRRYFDVWGAGTTVTVSS | 7 |
| | VL | DIQMTQSPSSLSASLGERVSLTCRASQDIGSNLNWLQLGPDG TIKRLIYATFSLDSGVPKRFSGSRSGSDYSLTISSLESEDFVDY YCQQYANFPPTFGGGTKLEIK | 8 |
| Chi-169 | VH | EVKLVESGGGLVKPGASLKLSCAASGFTFSNYGMSWVRQT SDKRLEWVASISSGGGRIYYPDNVKGRFTISRENAKNTLYLQ MSSLKSEDTALYYCTRHAMDNWGQGTSVIVSS | 15 |
| | VL | QIVLTQSPAIMSASPGEKVTMTCSASSSVSNMYWYQQKSGT SPKRWIYDTSKLASGVPARFSGSGSGTSYSLTISSMEAEDAS TYYCQQWSSNPLTFGAGTTLELK | 16 |
| Chi-89 | VH | EVQLVESGGDLVKPGGSLKLSCAASGFTFSSYGMSWVRQTP DKRLEWVATISSGGSYTYYPDSVKGRFTFSRDNAKNTLYLQ MSSLKSEDTAMYYCVRQALRYHMDSWGQGTSVTVSS | 23 |
| | VL | DIVMTQSQKFMSTSVGDRVSVTCKASQNVGTNVAWYQQK PGQSPKALIYSASYRYSGVPDRFTGSGSGTDFTLTISNVQSED LADFFCQQYYSYPWTFGGGTKLEIK | 24 |
| Chi-105 | VH | EVQLQQSGPELVKPGASVKMSCKASGYTFTDYYMNWVKQ | 31 |
| | | SHGKSLEWIGYIYPNNGGTGYNQRFKGKATLTADKSSSTAY MELRSLTSEDSAVYYCARWGGTRLGYYAMDYWGQGTSVT VSS | |
| | VL | DIVMTQSQKFMSTSVGDRVSVTCKASQNVGTNVVWYQQK PGQSPKLLIYWASTRHTGVPDRFTGSGSGTDFTLTISNVQSE DLADYFCQQYSRYPYTFGGGTKLEIK | 32 |
| Chi-128 | VH | DVQLVESGGGLVQPGGSRKVSCAASGFTFSSFGMHWVRQA PEKGLEWVAYISRGSSTIYYADTVKGRFTISRDNPKNTLFLQ MTSLRSEDTAMYYCARSGYGSSSYYFDYWGQGTTLTVSS | 39 |
| | VL | ENVLTQSPAIMSASPREKVTMTCRASSSIRSSYLHWYQQKS GASPKLWIYSTSNLASGVPARFSGSGSGTSYSLTISSVEAEDA ATYYCQQFSGYPLTFGGGTKLEIK | 40 |

### II. Antibody variant

In some embodiments, a variant of the anti-GPRCSD antibody or an antigen-binding fragment thereof is provided. The amino acid sequence variant of an anti-GPRCSD antibody may be prepared by introducing appropriate modifications to the nucleotide sequence encoding the anti-GPRCSD antibody or by peptide synthesis. Such modifications are, for example, deletions of amino acid residues in the amino acid sequence of the antibody, insertions of amino acid residues into the amino acid sequence, or/and substitutions of amino acid residues in the amino acid sequence. Any combination of deletions, insertions, and substitutions can be produced to give the final construct, so long as the final variant possesses the desired characteristics, e.g., antigen-binding effect, e.g., tumor killing efficacy, and the like.

### III. Fusion protein

In some embodiments, the present disclosure provides a fusion protein comprising the anti-GPRC5D antibody or the antigen-binding fragment thereof described herein.

### IV. Pharmaceutical composition

The present disclosure provides a pharmaceutical composition comprising the anti-GPRCSD antibody or the antigen-binding fragment thereof or the fusion protein, and further comprising a pharmaceutically acceptable carrier. In some embodiments, any one or more of antibodies Chi-89, Chi-105, Chi-128, Chi-131, Chi-164, Chi-169, and Chi-180, and a pharmaceutically acceptable carrier are formulated into the pharmaceutical composition. The pharmaceutically acceptable carrier includes, for example, excipients, diluents, encapsulating materials, fillers, buffering agents, or other agents.

### V. Isolated nucleic acid

The present disclosure provides an isolated nucleic acid encoding the anti-GPRCSD antibody or the antigen-binding fragment thereof described herein. In some embodiments, the nucleic acid encodes an antigen-binding fragment, e.g., the antigen-binding fragment of Chi-89, Chi-105, Chi-128, Chi-131, Chi-164, Chi-169, or Chi-180. In some embodiments, the nucleic acid encodes an antibody, e.g., Chi-89, Chi-105, Chi-128, Chi-131, Chi-164, Chi-169, and Chi-180. The nucleic acid sequences of some anti-GPRCSD antibodies or antigen-binding fragments thereof are illustratively listed in the sequence listing.

### VI. Vector

The present disclosure provides a vector comprising the isolated nucleic acid. In some embodiments, the vector is a cloning vector; in some other embodiments, the vector is an expression vector. The expression vector is optionally any expression vector capable of expressing the anti-GPRCSD antibody or the antigen-binding fragment thereof described herein. As one specific example, the expression vector is pcDNA3.1.

### VII. Host cell

In some embodiments, the present disclosure provides a host cell comprising the vector described herein or integrated in the genome with the nucleic acid. The host cell is a suitable host cell for cloning or expressing the anti-GPRCSD antibody or the antigen-binding fragment thereof. In some embodiments, the host cell is a prokaryotic cell. In some other embodiments, the host cell is a eukaryotic cell. In some embodiments, the host cell is selected from yeast cells, mammalian cells or other cells suitable for preparing the anti-GPRCSD antibody or the antigen-binding fragment thereof. The mammalian cells are, for example, Chinese hamster ovary (CHO) cells or CHO-S cells.

### VIII. Methods for preparing anti-GPRC5D antibody and antigen-binding fragment thereof

In some embodiments, the present disclosure provides a method for preparing the anti-GPRCSD antibody or the antigen-binding fragment thereof, comprising: culturing the host cell described herein, and isolating the anti-GPRCSD antibody or the antigen-binding fragment thereof from the host cell or the host cell culture.

To produce the anti-GPRCSD antibody or the antigen-binding fragment thereof, a nucleic acid encoding the anti-GPRCSD antibody or the antigen-binding fragment thereof is inserted into a vector for further cloning or/and expression in the host cell. The nucleic acid can be obtained using various methods known in the art, such as gene splicing and chemical synthesis. The isolation can be performed through procedures such as centrifugation or affinity chromatography.

### IX. Use

In some embodiments, the anti-GPRCSD antibody or the antigen-binding fragment thereof, and the fusion protein provided herein can be used for treating a cancer, reducing a tumor, or inhibiting tumor cell growth.

The present disclosure provides use of the anti-GPRCSD antibody or the antigen-binding fragment thereof or the fusion protein for preparing a medicament. In some embodiments, the use is for preparing a medicament for treating a disease. In some embodiments, the use is for preparing a medicament for reducing a tumor or inhibiting tumor cell growth.

The present disclosure provides a method for reducing or inhibiting tumor cell growth in a subject, comprising administering to the subject a therapeutically effective amount of the anti-GPRCSD antibody or the antigen-binding fragment thereof, the fusion protein, or the pharmaceutical composition.

The present disclosure provides a method for treating a disease in a subject, comprising administering to the subject a therapeutically effective amount of the anti-GPRCSD antibody or the antigen-binding fragment thereof, the fusion protein, or the pharmaceutical composition. Subjects in need of treatment include those with a disease or condition, as well as those who may have the disease or condition and whose purpose is to prevent, delay, or alleviate the disease or condition.

The disease described above is a cancer or an autoimmune disease. In some embodiments, the autoimmune disease is, for example, systemic lupus erythematosus and/or rheumatoid arthritis. In some embodiments, the cancer is multiple myeloma.

In some embodiments, a method for detecting or measuring GPRCSD in a sample is provided, comprising contacting the sample with the anti-GPRCSD antibody or the antigen-binding fragment thereof described herein, and detecting or measuring a binding complex.

### X. Kit

The present disclosure provides a kit comprising the anti-GPRCSD antibody or the antigen-binding fragment thereof, the fusion protein, or the pharmaceutical composition described herein.

A kit comprising the anti-GPRCSD antibody or the antigen-binding fragment thereof, the fusion protein, or the pharmaceutical composition is described herein. The kit may be used to implement the use of the anti-GPRCSD antibody or the antigen-binding fragment thereof, the fusion protein, or the pharmaceutical composition provided herein, or other use. In some embodiments, the kit may comprise the anti-GPRCSD antibody or the antigen-binding fragment thereof, the fusion protein, or the pharmaceutical composition described herein, and a reagent for detecting the presence of GPRCSD in a biological sample. The kit may further comprise an instruction for use. The kit may also comprise other materials as required from a commercial and user standpoint, for example, other buffers, diluents, needles, syringes, and the like.

The present disclosure further provides the following specific embodiments, which, however, are not intended to limit the scope of the present disclosure:
Embodiment 1. An isolated anti-GPRCSD antibody or an antigen-binding fragment thereof, wherein the anti-GPRCSD antibody comprises:
   a heavy chain variable region comprising:
   a HCDR1 comprising the amino acid sequence set forth in SEQ ID NO: 1, 9, 17, 25, or 33,
   a HCDR2 comprising the amino acid sequence set forth in SEQ ID NO: 2, 10, 18, 26, or 34, and
   a HCDR3 comprising the amino acid sequence set forth in SEQ ID NO: 3, 11, 19, 27, or 35; and
   a light chain variable region comprising:
      a LCDR1 comprising the amino acid sequence set forth in SEQ ID NO: 4, 12, 20, 28, or 36,
      a LCDR2 comprising the amino acid sequence set forth in SEQ ID NO: 5, 13, 21, 29, or 37, and
      a LCDR3 comprising the amino acid sequence set forth in SEQ ID NO: 6, 14, 22, 30, or 38.
Embodiment 2. The isolated anti-GPRC5D antibody or the antigen-binding fragment thereof according to embodiment 1, wherein the anti-GPRC5D antibody comprises:
   (i) a heavy chain variable region comprising:
      a HCDR1 comprising the amino acid sequence set forth in SEQ ID NO: 1,
      a HCDR2 comprising the amino acid sequence set forth in SEQ ID NO: 2, and
      a HCDR3 comprising the amino acid sequence set forth in SEQ ID NO: 3; and
      a light chain variable region comprising:
         a LCDR1 comprising the amino acid sequence set forth in SEQ ID NO: 4,
         a LCDR2 comprising the amino acid sequence set forth in SEQ ID NO: 5, and
         a LCDR3 comprising the amino acid sequence set forth in SEQ ID NO: 6;
   (ii) a heavy chain variable region comprising:
      a HCDR1 comprising the amino acid sequence set forth in SEQ ID NO: 9,
      a HCDR2 comprising the amino acid sequence set forth in SEQ ID NO: 10, and
      a HCDR3 comprising the amino acid sequence set forth in SEQ ID NO: 11; and
      a light chain variable region comprising:
         a LCDR1 comprising the amino acid sequence set forth in SEQ ID NO: 12,
         a LCDR2 comprising the amino acid sequence set forth in SEQ ID NO: 13, and
         a LCDR3 comprising the amino acid sequence set forth in SEQ ID NO: 14;
   (iii) a heavy chain variable region comprising:
      a HCDR1 comprising the amino acid sequence set forth in SEQ ID NO: 17,
      a HCDR2 comprising the amino acid sequence set forth in SEQ ID NO: 18, and
      a HCDR3 comprising the amino acid sequence set forth in SEQ ID NO: 19; and
      a light chain variable region comprising:
         a LCDR1 comprising the amino acid sequence set forth in SEQ ID NO: 20,
         a LCDR2 comprising the amino acid sequence set forth in SEQ ID NO: 21, and
         a LCDR3 comprising the amino acid sequence set forth in SEQ ID NO: 22;
   (iv) a heavy chain variable region comprising:
      a HCDR1 comprising the amino acid sequence set forth in SEQ ID NO: 25,
      a HCDR2 comprising the amino acid sequence set forth in SEQ ID NO: 26, and
      a HCDR3 comprising the amino acid sequence set forth in SEQ ID NO: 27; and
      a light chain variable region comprising:
         a LCDR1 comprising the amino acid sequence set forth in SEQ ID NO: 28,
         a LCDR2 comprising the amino acid sequence set forth in SEQ ID NO: 29, and
         a LCDR3 comprising the amino acid sequence set forth in SEQ ID NO: 30; or
   (v) a heavy chain variable region comprising:
      a HCDR1 comprising the amino acid sequence set forth in SEQ ID NO: 33,
      a HCDR2 comprising the amino acid sequence set forth in SEQ ID NO: 34, and
      a HCDR3 comprising the amino acid sequence set forth in SEQ ID NO: 35; and
      a light chain variable region comprising:
         a LCDR1 comprising the amino acid sequence set forth in SEQ ID NO: 36,
         a LCDR2 comprising the amino acid sequence set forth in SEQ ID NO: 37, and
         a LCDR3 comprising the amino acid sequence set forth in SEQ ID NO: 38.
Embodiment 3. An isolated anti-GPRCSD antibody or an antigen-binding fragment thereof, wherein the anti-GPRCSD antibody comprises a heavy chain variable region comprising the HCDR1, the HCDR2, and the HCDR3 of the variable region sequence set forth in SEQ ID NO: 7, 15, 23, 31, or 39, and a light chain variable region comprising the LCDR1, the LCDR2, and the LCDR3 of the variable region sequence set forth in SEQ ID NO: 8, 16, 24, 32, or 40. Embodiment 4. The anti-GPRCSD antibody or the antigen-binding fragment thereof according to embodiment 3, wherein the anti-GPRCSD antibody comprises a heavy chain variable region and a light chain variable region; the heavy chain variable region comprises the HCDR1, the HCDR2, and the HCDR3 of the variable region sequence set forth in SEQ ID NO: 7, and the light chain variable region comprises the LCDR1, the LCDR2, and the LCDR3 of the variable region sequence set forth in SEQ ID NO: 8; the heavy chain variable region comprises the HCDR1, the HCDR2, and the HCDR3 of the variable region sequence set forth in SEQ ID NO: 15, and the light chain variable region comprises the LCDR1, the LCDR2, and the LCDR3 of the variable region sequence set forth in SEQ ID NO: 16; the heavy chain variable region comprises the HCDR1, the HCDR2, and the HCDR3 of the variable region sequence set forth in SEQ ID NO: 23, and the light chain variable region comprises the LCDR1, the LCDR2, and the LCDR3 of the variable region sequence set forth in SEQ ID NO: 24; the heavy chain variable region comprises the HCDR1, the HCDR2, and the HCDR3 of the variable region sequence set forth in SEQ ID NO: 31, and the light chain variable region comprises the LCDR1, the LCDR2, and the LCDR3 of the variable region sequence set forth in SEQ ID NO: 32; or the heavy chain variable region comprises the HCDR1, the HCDR2, and the HCDR3 of the variable region sequence set forth in SEQ ID NO: 39, and the light chain variable region comprises the LCDR1, the LCDR2, and the LCDR3 of the variable region sequence set forth in SEQ ID NO: 40.
Embodiment 5. The isolated anti-GPRCSD antibody or the antigen-binding fragment thereof according to any one of embodiments 1 to 4, wherein the heavy chain variable region comprises an amino acid sequence having at least 80% identity to the sequence set forth in SEQ ID NO: 7, 15, 23, 31, or 39.
Embodiment 6. The isolated anti-GPRCSD antibody or the antigen-binding fragment thereof according to any one of embodiment 1 to 5, wherein the light chain variable region comprises an amino acid sequence having at least 80% identity to the sequence set forth in SEQ ID NO: 8, 16, 24, 32, or 40.
Embodiment 7. The isolated anti-GPRCSD antibody or the antigen-binding fragment thereof according to any one of embodiment 1 to 6, wherein the heavy chain variable region comprises an amino acid sequence having at least 80% identity to the sequence set forth in SEQ ID NO: 7, 15, 23, 31, or 39, and the light chain variable region comprises an amino acid sequence having at least 80% identity to the sequence set forth in SEQ ID NO: 8, 16, 24, 32, or 40. Embodiment 8. The isolated anti-GPRCSD antibody or the antigen-binding fragment thereof according to embodiment 7, wherein the heavy chain variable region and the light chain variable region are selected from any one of the following:
   (i) the heavy chain variable region comprises an amino acid sequence having at least 80% identity to the sequence set forth in SEQ ID NO: 7, and the light chain variable region comprises an amino acid sequence having at least 80% identity to the sequence set forth in SEQ ID NO: 8;
   (ii) the heavy chain variable region comprises an amino acid sequence having at least 80% identity to the sequence set forth in SEQ ID NO: 15, and the light chain variable region comprises an amino acid sequence having at least 80% identity to the sequence set forth in SEQ ID NO: 16;
   (iii) the heavy chain variable region comprises an amino acid sequence having at least 80% identity to the sequence set forth in SEQ ID NO: 23, and the light chain variable region comprises an amino acid sequence having at least 80% identity to the sequence set forth in SEQ ID NO: 24;
   (iv) the heavy chain variable region comprises an amino acid sequence having at least 80% identity to the sequence set forth in SEQ ID NO: 31, and the light chain variable region comprises an amino acid sequence having at least 80% identity to the sequence set forth in SEQ ID NO: 32; or
   (v) the heavy chain variable region comprises an amino acid sequence having at least 80% identity to the sequence set forth in SEQ ID NO: 39, and the light chain variable region comprises an amino acid sequence having at least 80% identity to the sequence set forth in SEQ ID NO: 40.
Embodiment 9. The isolated anti-GPRCSD antibody or the antigen-binding fragment thereof according to embodiment 7, wherein the heavy chain variable region and the light chain variable region are selected from any one of the group consisting of:
   (i) the heavy chain variable region comprises the amino acid sequence set forth in SEQ ID NO: 7, and the light chain variable region comprises the amino acid sequence set forth in SEQ ID NO: 8;
   (ii) the heavy chain variable region comprises the amino acid sequence set forth in SEQ ID NO: 15, and the light chain variable region comprises the amino acid sequence set forth in SEQ ID NO: 16;
   (iii) the heavy chain variable region comprises the amino acid sequence set forth in SEQ ID NO: 23, and the light chain variable region comprises the amino acid sequence set forth in SEQ ID NO: 24;
   (iv) the heavy chain variable region comprises the amino acid sequence set forth in SEQ ID NO: 31, and the light chain variable region comprises the amino acid sequence set forth in SEQ ID NO: 32; or
   (v) the heavy chain variable region comprises the amino acid sequence set forth in SEQ ID NO: 39, and the light chain variable region comprises the amino acid sequence set forth in SEQ ID NO: 40.
Embodiment 10. The isolated anti-GPRCSD antibody or the antigen-binding fragment thereof according to any one of embodiments 1 to 9, wherein the antigen-binding fragment is an Fab fragment, an Fab' fragment, an F(ab')₂ fragment, an Fd fragment, an Fv fragment, an isolated CDR region, or an scFv.
Embodiment 11. The isolated anti-GPRCSD antibody or the antigen-binding fragment thereof according to any one of embodiments 1 to 10, wherein the anti-GPRCSD antibody is a chimeric, humanized, or fully human antibody.
Embodiment 12. The isolated anti-GPRCSD antibody or the antigen-binding fragment thereof according to any one of embodiments 1 to 11, wherein the anti-GPRC5D antibody is of the IgG1, IgG2, IgG3, or IgG4 isotype.
Embodiment 13. The isolated anti-GPRCSD antibody or the antigen-binding fragment thereof according to any one of embodiments 1 to 12, wherein the anti-GPRCSD antibody or the antigen-binding fragment thereof binds to a human GPRCSD antigen and optionally, further features any one or more of the following properties:
   (1) binding to a monkey GPRCSD antigen;
   (2) binding to a mouse GPRCSD antigen;
   (3) no binding to one or more of GPRCSA, GPRCSB, and GPRCSC.
Embodiment 14. The isolated anti-GPRCSD antibody or the antigen-binding fragment thereof according to any one of embodiments 1 to 13, wherein the anti-GPRCSD antibody is a monospecific antibody or a multispecific antibody.
Embodiment 15. A fusion protein comprising the anti-GPRCSD antibody or the antigen-binding fragment thereof according to any one of embodiments 1 to 14.
Embodiment 16. A pharmaceutical composition comprising the anti-GPRCSD antibody or the antigen-binding fragment thereof according to any one of embodiments 1 to 14 or the immunoconjugate according to embodiment 15 and further comprising a pharmaceutically acceptable carrier.
Embodiment 17. An isolated nucleic acid encoding the anti-GPRCSD antibody or the antigen-binding fragment thereof according to any one of embodiments 1 to 14.
Embodiment 18. A vector, comprising the nucleic acid according to embodiment 17.
Embodiment 19. A host cell, comprising the vector according to embodiment 18 or integrated in the genome with the nucleic acid according to embodiment 17.
Embodiment 20. A method for preparing the anti-GPRCSD antibody or the antigen-binding fragment thereof according to any one of embodiments 1 to 14, comprising: culturing the host cell according to embodiment 19, and isolating the anti-GPRCSD antibody or the antigen-binding fragment thereof from the host cell or the host cell culture.
Embodiment 21. The anti-GPRCSD antibody or the antigen-binding fragment thereof according to any one of embodiments 1 to 14, the fusion protein according to embodiment 15, or the pharmaceutical composition according to embodiment 16 for treating a disease, or reducing a tumor or inhibiting tumor cell growth, wherein preferably the disease is a cancer or an autoimmune disease.
Embodiment 22. Use of the anti-GPRCSD antibody or the antigen-binding fragment thereof according to any one of embodiments 1 to 14 or the fusion protein according to embodiment 15 for preparing a medicament for treating a disease, reducing a tumor, or inhibiting tumor cell growth, wherein preferably, the disease is a cancer or an autoimmune disease.
Embodiment 23. A method for reducing a tumor or inhibiting tumor cell growth in a subject, comprising administering to the subject a therapeutically effective amount of the anti-GPRC5D antibody or the antigen-binding fragment thereof according to any one of embodiments 1 to 14, the fusion protein according to embodiment 15, or the pharmaceutical composition according to embodiment 16.
Embodiment 24. A method for treating a disease in a subject, comprising administering to the subject a therapeutically effective amount of the anti-GPRCSD antibody or the antigen-binding fragment thereof according to any one of embodiments 1 to 14, the fusion protein according to embodiment 15, or the pharmaceutical composition according to embodiment 16, wherein preferably, the disease is a cancer or an autoimmune disease.
Embodiment 25. The embodiment according to any one of embodiments 21, 22, and 24, wherein the cancer is multiple myeloma.
Embodiment 26. A kit, comprising the anti-GPRCSD antibody or the antigen-binding fragment thereof according to any one of embodiments 1 to 14, the fusion protein according to embodiment 15, or the pharmaceutical composition according to embodiment 16.

### Examples

### Example 1: Antigen immunization

### 1. DNA antigen immunization

A cDNA sequence encoding human GPRCSD protein (with the amino acid sequence set forth in SEQ ID NO: 41) was obtained by gene synthesis and subcloned into expression vector pcDNA3.1(+) to construct a plasmid pcDNA3.1-hGPRCSD. A large quantity of plasmid was prepared according to the instructions for EndoFree Plasmid Giga Kit (QIAGEN, Cat. No.: 12391).

The plasmid pcDNA3.1-hGPRCSD was used as the antigen to immunize A/J mice (Model Animal Research Center of Nanjing University), BALB/c mice (Shanghai Lingchang), and SJL mice (Beijing Vital River) separately. Firstly, hyaluronidase (Sigma, Cat. No.: H4272) was pre-injected into the left and right hind limb muscles of each mouse for pretreatment. Then, CpG (InvivoGen, Cat. No.: tlrl-1826) and the plasmid pcDNA3.1-hGPRCSD were mixed well in a mass ratio of 1:1, and the well-mixed antigen complex was injected into the pretreated site of the muscle in the mice with an *in vivo* gene transfer instrument (Shanghai Teresa Biotechnology Co., Ltd., type II). The immunization procedures described above were repeated once every 2 to 3 weeks for a total of 4 immunizations. After the completion of the immunizations, the serum was collected from each mouse.

### 2. Mouse serum titer assessment

The antibody titer in the mouse serum was detected by FACS to assess the immune response of anti-human GPRCSD antibodies produced in mice immunized with DNA antigens. Mice with a higher serum titer were selected for intensive immunization before subsequent splenocyte fusion.

### 3. Cell antigen immunization

The plasmid pcDNA3.1-hGPRCSD was transfected into CHO-K1 cells according to the instructions for lipofectamine3000 transfection reagent (Thermo, Cat. No.: L3000015) to give a cell line CHO-K1-hGPRC5D^{hi} with stable high expression of hGPRC5D. Mice were subjected to intensive immunization with the CHO-K1-hGPRC5D^{hi} cell described above as the antigen: 3-4 days before splenocyte fusion, a CHO-K1-hGPRC5D^{hi} cell suspension at a final cell concentration of 5 × 10⁷ cells/mL and CpG (InvivoGen, Cat. No.: tlrl-1826) at a final concentration of 0.5 mg/mL were mixed well, and then intraperitoneally injected into the mice at 100 µL/mouse.

### Example 2: Screening of mouse anti-human GPRC5D antibodies

### 1. Preparation of hybridoma cells

On the day of fusion, the mouse spleen was collected aseptically and ground before a red cell lysis buffer (Sigma, Cat. No.: R7757) was added. After the cells were washed with a PBS solution, the splenocytes were resuspended in an electrofusion buffer (BTX, Cat. No.: 47-0001) and mixed well with mouse myeloma cells SP2/0 in a cell number ratio of 2:1. The splenocyte fusion was performed on an electrofusion system (BTX). The fused cells were diluted by adding a hybridoma medium (Gibco, Cat. No.: 12045-076) containing 1× HAT (Gibco, Cat. No.: 21060-017), and then cultured at 37 °C/5% CO₂ for 7-10 days to prepare hybridoma cells. The hybridoma cell culture supernatant was collected for screening of mouse anti-human GPRCSD antibodies.

### 2. Construction of stable cell lines

The following cell lines were constructed to assess the species cross-binding activity of the mouse anti-human GPRCSD antibodies at the cellular level: cDNAs encoding full-length monkey GPRCSD (with amino acid sequence set forth in SEQ ID NO: 42) and mouse GPRCSD (with amino acid sequence set forth in SEQ ID NO: 43) were obtained by gene synthesis, and then subcloned into a vector pcDNA3.1(+) to give pcDNA3.1-cynoGPRCSD and pcDNA3.1-murineGPRC5D recombinant plasmids. Plasmids pcDNA3.1-cynoGPRCSD and pcDNA3.1-murineGPRC5D were separately transfected into CHO-K1 cells according to the instructions for a lipofectamine3000 transfection reagent (Thermo, Cat. No.: L3000015) to give stable cell lines CHO-K1-cynoGPRC5D and CHO-K1-murineGPRC5D.

### 3. Preliminary cell screening by ELISA

CHO-K1-hGPRC5D^{hi} cells in the logarithmic growth phase were collected and resuspended in a DMEM/F-12 medium (Gibco, Cat. No.: 11320033) containing 10% (by volume) of FBS. The cell concentration was adjusted to 1 × 10⁶ cells/mL. The cells were plated into a 96-well flat bottom plate at 100 µL/well, and then incubated overnight at 37 °C/5% CO₂. On the next day, the cell plate was washed with PBS before 4% (by volume) Paraformaldehyde Fix Solution (Beyotime, Cat. No.: P0099) was added, and let stand at room temperature for 30 min. After the plate was washed with PBS, a PBS solution containing 3% (by volume) of BSA was added at 200 µL/well. The mixture was blocked at room temperature for 2 h. After blocking, the hybridoma cell culture supernatant was added to the cell plate at 100 µL/well, and the mixture was incubated at room temperature for 2 h. The plate was washed with PBS. Then, an HRP-conjugated goat anti-mouse IgG+IgM(H+L) antibody (Jackson Immuno Research, Cat. No.: 115-035-068) was added, and the mixture was incubated at room temperature for 1 h. After the plate was washed with PBS, a TMB reaction solution (Solarbio, Cat. No.: RP1200) was added at 100 µL/well, and the mixture was incubated at room temperature in the dark for 5 min. The reaction was stopped with 0.5 M H₂SO₄. The OD450 absorbance was read on a Bio-rad iMark microplate reader. Hybridoma cells corresponding to the culture supernatant with an absorbance greater than 1.0 were selected as the positive clones for subsequent screening by FACS.

### 4. Antibody screening by flow cytometry FACS

A CHO-K1-cynoGPRC5D cell suspension at a cell concentration of 5 × 10⁵ cells/mL was plated into a 96-well U-bottom plate at 100 µL/well. The culture supernatant of the positive clones was added at 100 µL/well. The mixture was mixed well, and then incubated at 4 °C for 1 h. The cells were washed with PBS containing 2% (by volume) of FBS. An AlexaFluor488-conjugated goat anti-mouse IgG+IgM(H+L) antibody (Jackson Immuno Research, Cat. No.: 115-545-044) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to CHO-K1-cynoGPRC5D cells was analyzed by the mean fluorescence intensity (MFI) of staining.

### 5. Preparation of subclones

Hybridoma cells that were positively for binding to both CHO-K1-hGPRC5D^{hi} and CHO-K1-cynoGPRC5D after the cell screening by ELISA and FACS were selected for expansion culture. The expanded cells were mixed well with a semi-solid medium D (Stemcell, Cat. No.: 3810) in a volume ratio of 1:10, and then incubated at 37 °C/5% CO₂ for 5 days. The monoclonal cell mass was pipetted under 4-fold microscopy and transferred to a 96-well plate plated with a hybridoma medium (Gibco, Cat. No.: 12045-076). Then the monoclonal cell mass was placed at 37 °C/5% CO₂ for 2 days to continue the culture. The subcloned cell culture supernatant of each well was collected for further screening of subclones.

### 6. Screening of subclones

### (1) Screening of anti-hGPRCSD antibodies by FACS

An H929 (i.e., NCI-H929) cell suspension at a cell concentration of 3 × 10⁵ cells/mL was plated into a 96-well U-bottom plate at 100 µL/well. The subcloned cell culture supernatant was added at 100 µL/well or an equal volume of culture medium (hereinafter referred to as Medium) was added. The mixture was mixed well, and then incubated at 4 °C for 1 h. The cells were washed with PBS containing 2% (by volume) of FBS. An AlexaFluor488-conjugated goat anti-mouse IgG+IgM(H+L) antibody (Jackson Immuno Research, Cat. No.: 115-545-044) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the mouse anti-hGPRCSD antibody to H929 cells was analyzed by the mean fluorescence intensity (MFI) of staining. Part of the results are shown in Table 1.

**Table 1. Binding assay of mouse anti-hGPRC5D antibodies to H929 cells by FACS**

| **Clone No.** | **H929MFI** | **Clone No.** | **H929MFI** | **Clone No.** | **H929MFI** |
|---|---|---|---|---|---|
| mu-7 | 53151 | mu-74.3 | 18078 | mu-131 | 12826 |
| mu-9 | 10704 | mu-89 | 11919 | mu-153 | 46867 |
| mu-45 | 21606 | mu-105 | 13248 | mu-164 | 14184 |
| mu-38.1 | 75956 | mu-125 | 11670 | mu-169 | 59318 |
| mu-35 | 54719 | mu-126 | 10271 | mu-180 | 26579 |
| mu-51 | 95531 | mu-128 | 15661 | mu-183 | 27008 |
| mu-58 | 464150 | mu-129 | 7904 | Medium | 8242 |

### (2) Cell ELISA screening of anti-hGPRC5D antibodies

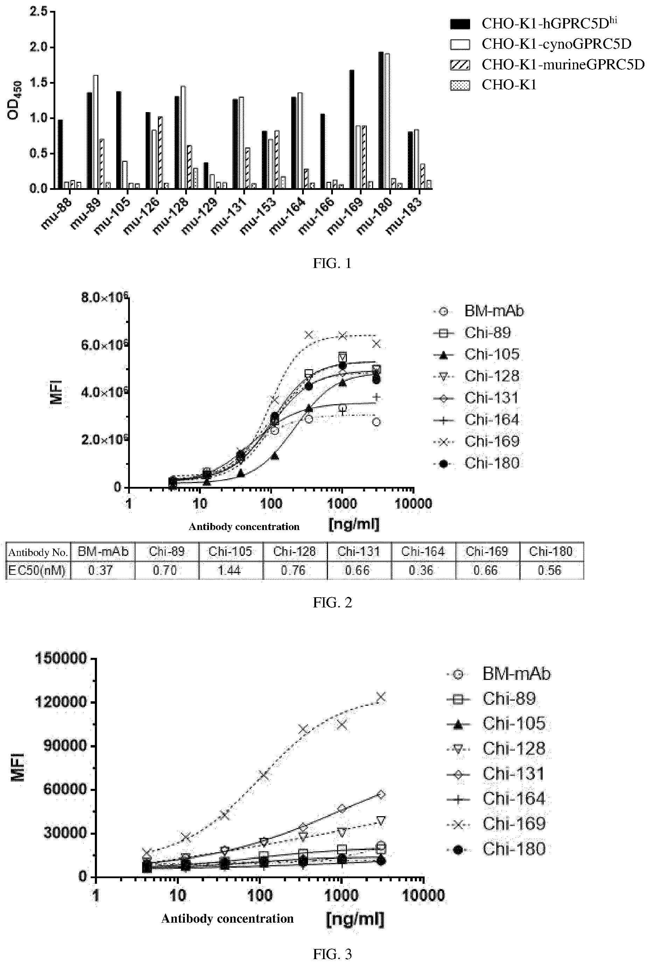

CHO-K1-hGPRC5D^{hi} cells, CHO-K1-cynoGPRC5D cells, CHO-K1-murineGPRC5D cells, and CHO-K1 cells in the logarithmic growth phase were separately collected and resuspended in a DMEM/F-12 (Gibco, Cat. No.: 11320033) medium containing 10% (by volume) of FBS. The cell concentration was adjusted to 1 × 10⁶ cells/mL. The cells were plated into a 96-well flat bottom plate at 100 µL/well, and then incubated overnight at 37 °C/5% CO₂. On the next day, the cell plate was washed with PBS before 4% (by volume) Paraformaldehyde Fix Solution (Beyotime, Cat. No.: P0099) was added, and let stand at room temperature for 30 min. After the plate was washed with PBS, a PBS solution containing 3% (by volume) of BSA was added at 200 µL/well. The mixture was blocked at room temperature for 2 h. After blocking, the subcloned cell culture supernatant was added to each cell plate at 50 µL/well, and the mixture was incubated at room temperature for 2 h. The plate was washed with PBS. Then, an HRP-conjugated goat anti-mouse IgG+IgM(H+L) antibody (Jackson Immuno Research, Cat. No.: 115-035-068) was added, and the mixture was incubated at room temperature for 1 h. After the plate was washed with PBS, a TMB reaction solution (Solarbio, Cat. No.: RP1200) was added at 100 µL/well, and the mixture was incubated at room temperature in the dark for 5 min. The reaction was stopped with 0.5 M H₂SO₄. The OD450 absorbance was read on a Bio-rad iMark microplate reader. A subclone absorbance histogram was generated using Graphpad Prism, as shown in FIG. 1 for typical mouse anti-hGPRCSD antibodies (e.g., mu-89, mu-105, mu-126, mu-128, mu-131, mu-153, mu-164, mu-169, mu-180, and mu-183, etc.) that had at least a 2-fold difference in absorbance against CHO-K1-hGPRC5D^{hi} cells, CHO-K1-cynoGPRC5D cells, and (or) CHO-K1-murineGPRC5D cells as compared to the control cell CHO-K1.

### Example 3: Preparation of anti-hGPRC5D chimeric antibodies

### 1. Sequencing of variable regions of mouse anti-hGPRC5D antibodies

The total RNA of the hybridoma cells selected was isolated according to the instructions for an RNA extraction kit (Takara, Cat. No.: 9767), and a first-strand cDNA was synthesized by a reverse transcription kit (Thermo, Cat. No.: K1652). The first-strand cDNA was used as the template, and mixed with a mouse heavy chain constant region primer and a light chain constant region primer separately. The cDNA was then cloned and sequenced by polymerase chain reaction (PCR) to give the sequences of the variable regions of the mouse anti-hGPRCSD antibodies.

### 2. Construction of expression vectors of chimeric antibodies

The nucleotide sequences of the heavy chain variable region (VH) and the light chain variable region (VL) of the mouse anti-hGPRCSD antibodies were linked to the nucleotide sequences of a human IgG1 heavy chain constant region and a Kappa light chain constant region, respectively, by chemical synthesis. Recombinant human-mouse chimeric antibody expression vectors were constructed using a pcDNA3.1(+) vector for transfection and expression to prepare the chimeric antibodies. The nucleotide sequences and amino acid sequences of the full length and VH/VL of the chimeric antibodies are detailed in Table 2.

**Table 2. Nucleotide sequences and amino acid sequences of full length and VH/VL of chimeric antibodies**

| **Clone No.** | **Heavy chain amino acid/nucleotide sequence** | **VH amino acid sequence** | **Light chain amino acid/nucleotide sequence** | **VL amino acid sequence** |
|---|---|---|---|---|
| **Chi-89** | SEQ ID NO:49/50 | SEQ ID NO:23 | SEQ ID NO:51/52 | SEQ ID NO:24 |
| **Chi-105** | SEQ ID NO:53/54 | SEQ ID NO:31 | SEQ ID NO:55/56 | SEQ ID NO:32 |
| **Chi-128** | SEQ ID NO:57/58 | SEQ ID NO:39 | SEQ ID NO:59/60 | SEQ ID NO:40 |
| **Chi-131** | SEQ ID NO:61/62 | SEQ ID NO:7 | SEQ ID NO:63/64 | SEQ ID NO:8 |
| **Chi-169** | SEQ ID NO:65/66 | SEQ ID NO:15 | SEQ ID NO:67/68 | SEQ ID NO:16 |

### 3. Construction of expression vector of positive reference antibody

GC5B596 was selected as the reference antibody (a monoclonal antibody from WO2018017786A2). The nucleotide sequences of the VH (SEQ ID NO: 44) and VL (SEQ ID NO: 45) of the antibody were obtained by chemical synthesis, and were separately linked to the nucleotide sequences of a human IgG1 heavy chain constant region and a Kappa light chain constant region. An expression vector expressing the positive reference antibody was constructed using a pcDNA3.1(+) vector for transfection and expression to prepare the positive reference antibody. The positive reference antibody (monoclonal antibody) is designated as BM-mAb herein.

### 4. Preparation of anti-hGPRCSD chimeric antibody and positive reference antibody

The anti-hGPRCSD antibody was transiently transfected into cells according to the instruction manual of the expiCHO expression system (Gibco, Cat. No.: A29129). The transfected expiCHO cells were cultured at 37 °C/8% CO₂ for 7 days while shaking. The cell culture supernatant was collected, and the clear culture supernatant was loaded onto a protein A column (G.E.Healthcare, Cat. No.: 17-5474). The protein A column was washed with 10 column volumes of PBS buffer. The IgG antibodies bound to the protein A column were eluted with an acetic acid buffer (300 mM acetic acid, pH 3.6) and collected. The collected IgG antibody components were transferred into the PBS buffer by an ultrafiltration device (molecular weight cut-off 30 kDa, Millipore, Cat. No.: UFC903024) to give anti-hGPRCSD antibody solutions.

### Example 4: Binding activity assay for anti-hGPRC5D antibodies to cells stably expressing human GPRC5D

### 1. Binding properties of anti-hGPRC5D chimeric antibodies to cells with stable high expression of human GPRCSD

A CHO-K1-hGPRC5D^{hi} (with a GPRCSD expression level of about 2E+06 to 3E+06 antigens/cells) cell suspension at a cell concentration of 3 × 10⁵ cells/mL was plated in a 96-well U-bottom plate at 100 µL/well. Serially diluted anti-hGPRCSD antibodies (in a final concentration range of 4.1-3000 ng/mL, serially 3-fold diluted) were added. The mixture was mixed well and then incubated at 4 °C for 1 h. The cells were washed with PBS containing 2% (by volume) of FBS. A PE-conjugated goat anti-human IgG Fcy antibody (Jackson Immuno Research, Cat. No.: 109-116-170) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to CHO-K1-hGPRC5D^{hi} cells was analyzed by the mean fluorescence intensity (MFI) of staining. The analysis result of EC₅₀ calculated by GraphpadPrism is shown in FIG. 2. The chimeric antibodies Chi-89, Chi-105, Chi-128, Chi-131, Chi-164, Chi-169, and Chi-180 all exhibited binding activity in a concentration gradient-dependent manner on cells with high expression of hGPRCSD, and had higher maximum binding amounts to antigens than BM-mAb.

### 2. Binding properties of anti-hGPRC5D chimeric antibodies to cells with stable low expression of human GPRCSD

The plasmid pcDNA3.1-hGPRCSD was transfected into CHO-K1 cells according to the instructions for lipofectamine3000 transfection reagent (Thermo, Cat. No.: L3000015) to give a cell line CHO-K1-hGPRC5D^{low} with stable low expression (GPRCSD expression level of about 2000-2500 antigens/cells). A CHO-K1-hGPRC5D^{low} cell suspension at a cell concentration of 3 × 10⁵ cells/mL was plated in a 96-well U-bottom plate at 100 µL/well. Serially diluted anti-hGPRCSD antibodies (in a final concentration range of 4.1-3000 ng/mL, serially 3-fold diluted) were added. The mixture was mixed well and then incubated at 4 °C for 1 h. The cells were washed with PBS containing 2% (by volume) of FBS. A PE-conjugated goat anti-human IgG Fcy antibody (Jackson Immuno Research, Cat. No.: 109-116-170) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to CHO-K1-hGPRC5D^{low} cells was analyzed by the mean fluorescence intensity (MFI) of staining. As shown in FIG. 3, on the cells with low expression of hGPRCSD, the chimeric antibodies Chi-89, Chi-105, Chi-164, and Chi-180 showed comparable binding levels with BM-mAb, while Chi-128, Chi-131, and Chi-169 exhibited stronger target-binding ability.

### Example 5: Binding activity assay for anti-hGPRC5D antibodies to tumor cells

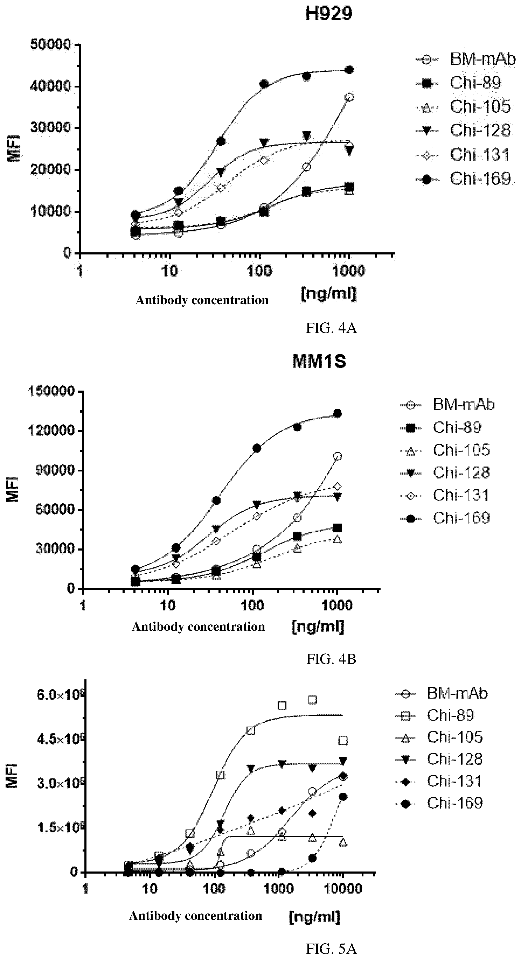

H929 and MM1S human myeloma cell suspensions at a cell concentration of 3 × 10⁵ cells/mL were separately plated in 96-well U-bottom plates at 100 µL/well. Serially diluted anti-hGPRCSD antibodies (in a final concentration range of 4.1-1000 ng/mL, serially 3-fold diluted) were added. The mixture was mixed well and then incubated at 4 °C for 1 h. The cells were washed with PBS containing 2% (by volume) of FBS. A PE-conjugated goat anti-human IgG Fcy antibody (Jackson Immuno Research, Cat. No.: 109-116-170) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to human myeloma cells was analyzed by the mean fluorescence intensity (MFI) of staining. The chimeric antibodies Chi-89, Chi-105, Chi-128, Chi-131, and Chi-169 all exhibited binding activity in a concentration gradient-dependent manner on both H929 and MM1S cells as shown in FIGs. 4A and 4B plotted using Graphpad Prism.

### Example 6: Species cross-activity assay for anti-hGPRC5D antibodies

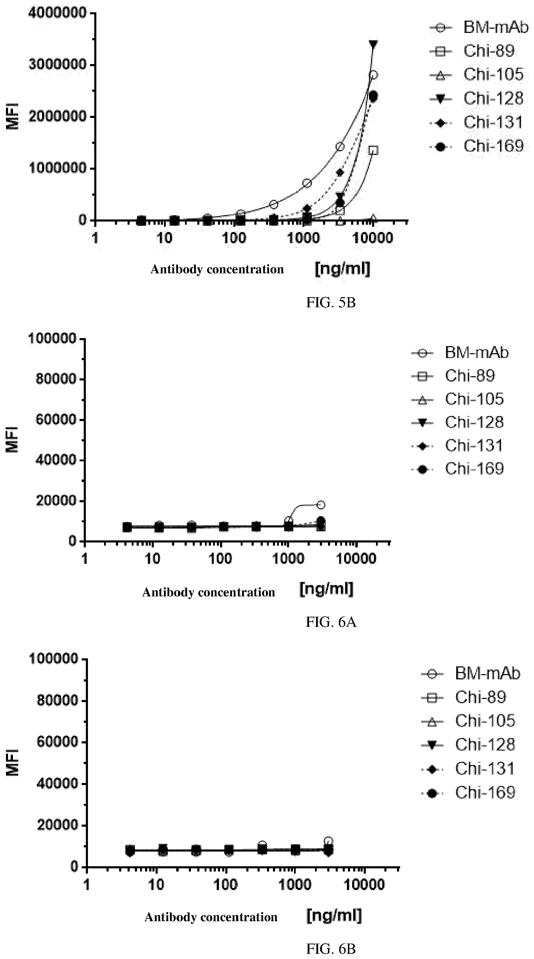

CHO-K1-cynoGPRC5D and CHO-K1-murineGPRC5D cell suspensions at a cell concentration of 5 × 10⁵ cells/mL were separately plated in 96-well U-bottom plates at 100 µL/well. Serially diluted anti-hGPRC5D antibodies (in a final concentration range of 4.6-10000 ng/mL, serially 3-fold diluted) were added. The mixture was mixed well and then incubated at 4 °C for 1 h. The cells were washed with PBS containing 2% (by volume) of FBS. A PE-conjugated goat anti-human IgG Fcy antibody (Jackson Immuno Research, Cat. No.: 109-116-170) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to CHO-K1-cynoGPRC5D and CHO-K1-murineGPRC5D cells was analyzed by the mean fluorescence intensity (MFI) of staining. As shown in FIGs. 5A and 5B, the chimeric antibodies Chi-89, Chi-128, Chi-131, and Chi-169 all exhibited binding activity in a concentration gradient-dependent manner on CHO-K1-cynoGPRC5D and CHO-K1-murineGPRC5D cells, indicating that these anti-hGPRCSD antibodies have cross-binding activity to monkey and mouse GPRCSD compared with BM-mAb, while chimeric antibody Chi-105 has cross-binding activity to monkey GPRCSD only.

### Example 7: Verification of specificity of anti-hGPRC5D antibodies

1. Verification of binding of anti-hGPRC5D chimeric antibodies to other family member proteins at cellular level It is known that the GPRC family include GPRCSA (RAIG1), GPRCSB (RAIG2), and GPRC5C (RAIG3) in addition to GPRCSD. cDNAs encoding full-length GPRCSA (SEQ ID NO: 46), GPRC5B (SEQ ID NO: 47), and GPRC5C (SEQ ID NO: 48) were obtained by gene synthesis, and then subcloned into a vector pcDNA3.1(+) to give pcDNA3.1-GPRCSA-GFP, pcDNA3.1-GPRCSB-GFP, and pcDNA3.1-GPRCSC-GFP recombinant plasmids. The DNAs of the recombinant plasmids described above were separately transfected into CHO-K1 cells according to the instructions for lipofectamin3000 transfection reagent (Thermo, Cat. No.: L3000015) to give stable cell lines CHO-K1-GPRC5A, CHO-K1-GPRC5B, and CHO-K1-GPRC5C.

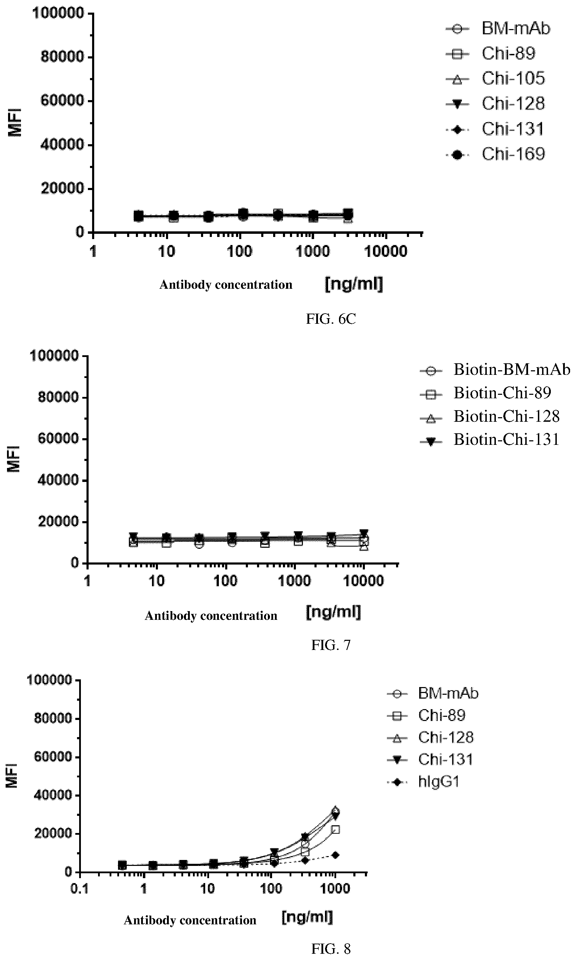

CHO-K1-GPRC5A, CHO-K1-GPRC5B, and CHO-K1-GPRC5C cell suspensions at a cell concentration of 5 × 10⁵ cells/mL were separately plated in 96-well U-bottom plates at 100 µL/well. Serially diluted anti-hGPRCSD antibodies (in a final concentration range of 4.1-3000 ng/mL, serially 3-fold diluted) were added. The mixture was mixed well and then incubated at 4 °C for 1 h. The cells were washed with PBS containing 2% (by volume) of FBS. An APC-conjugated goat anti-human IgG Fcy antibody (Jackson Immuno Research, Cat. No.: 109-135-098) was added, and the mixture was incubated at 4 °C for 1 h. The cells were washed with PBS containing 2% (by volume) of FBS and then resuspended. Fluorescence signals were detected on a flow cytometer (Sartorius, IQue3). The binding of the anti-hGPRCSD antibodies to CHO-K1-GPRC5A, CHO-K1-GPRC5B, and CHO-K1-GPRC5C cells was analyzed by the mean fluorescence intensity (MFI) of staining. As shown in FIGs. 6A, 6B, and 6C, the chimeric antibodies Chi-89, Chi-105, Chi-128, Chi-131, and Chi-169 exhibited no binding activity to CHO-K1-GPRC5A, CHO-K1-GPRC5B, and CHO-K1-GPRC5C cells in the concentration range of 4.1-3000 ng/mL, and the BM-mAb exhibited non-specific binding to CHO-K1-GPRC5A cells at concentrations of greater than 1000 ng/mL.

### 2. Verification of binding of anti-hGPRC5D chimeric antibodies to CHO-K1 cells

Firstly, biotin-conjugated anti-hGPRCSD antibodies were prepared according to the instructions for EZ-linkNHS-Biotin reagent (Thermo, Cat. No.: 20217), designated as Biotin-BM-mAb, Biotin-Chi-89, Biotin-Chi-128, and Biotin-Chi-131, respectively.

A CHO-K1 cell suspension at a cell concentration of 3 × 10⁵ cells/mL was plated in a 96-well U-bottom plate at 100 µL/well. Serially diluted biotin-conjugated anti-hGPRCSD antibodies (serially 3-fold diluted, in a final concentration range of 4.6-10000 ng/mL,) were added. The mixture was mixed well and then incubated at 4 °C for 1 h. The cells were washed with PBS containing 2% (by volume) of FBS. A PE-conjugated streptavidin (BD, Cat. No.: 554061) was added, and the mixture was incubated at 4 °C for 1 h. The cells were washed with PBS containing 2% (by volume) of FBS and then resuspended. Fluorescence signals were detected on a flow cytometer (Sartorius, IQue3). The mean fluorescence intensity (MFI) of staining was used to analyze whether the biotin-conjugated anti-hGPRCSD antibodies with amplified MFI signal exhibited non-specific binding to CHO-K1 cells. The results are shown in FIG. 7. The biotin-conjugated chimeric antibodies Chi-89, Chi-128, and Chi-131 all produced non-binding signals to CHO-K1 cells.

### Example 8: Internalizing activity of anti-hGPRC5D antibodies

MM1S cells were resuspended in an RPMI1640 medium (Gibco, Cat. No.: 22400071) containing 10% (by volume) of FBS. The cell concentration was adjusted to 2 × 10⁶ cells/mL. Then the cells were plated into a 96-well V-bottom plate at 20 µL/well. An antibody internalization reagent (Sartorius, Cat. No.: 90564) was added to anti-hGPRC5D antibodies BM-mAb, Chi-89, Chi-128, and Chi-131, and an isotype control antibody hIgG 1 (Biointron, Cat. No.: B117901). The mixture was incubated at 37 °C in the dark for 15 min. Then, the antibodies described above were serially diluted with an RPMI1640 medium (Gibco, Cat. No.: 22400071) containing 10% (by volume) of FBS (in a final concentration range of 0.46-1000 ng/mL, serially 3-fold diluted). The mixture was pipetted at 20 µL/well, mixed well with the cell suspension, and incubated at 37 °C for 2 h. After the incubation was completed, the cells were washed with PBS containing 2% (by volume) of FBS and then resuspended. Fluorescence signals were detected on a flow cytometer (Sartorius, IQue3). The internalizing activity of the anti-hGPRCSD antibodies was analyzed by mean fluorescence intensity (MFI) of staining. The results are shown in FIG. 8. The internalization of target GPRCSD mediated by chimeric antibodies Chi-89, Chi-128, and Chi-131 is comparable to that by BM-mAb, resulting in only weak internalization.

## Claims

1. An isolated anti-GPRC5D antibody or an antigen-binding fragment thereof, wherein the anti-GPRC5D antibody comprises:
a heavy chain variable region comprising:
a HCDR1 comprising the amino acid sequence set forth in SEQ ID NO: 1, 9, 17, 25, or 33,
a HCDR2 comprising the amino acid sequence set forth in SEQ ID NO: 2, 10, 18, 26, or 34, and
a HCDR3 comprising the amino acid sequence set forth in SEQ ID NO: 3, 11, 19, 27, or 35; and
a light chain variable region comprising:
a LCDR1 comprising the amino acid sequence set forth in SEQ ID NO: 4, 12, 20, 28, or 36,
a LCDR2 comprising the amino acid sequence set forth in SEQ ID NO: 5, 13, 21, 29, or 37, and
a LCDR3 comprising the amino acid sequence set forth in SEQ ID NO: 6, 14, 22, 30, or 38.

2. The isolated anti-GPRC5D antibody or the antigen-binding fragment thereof according to claim 1, wherein the anti-GPRC5D antibody comprises:
(i) a heavy chain variable region comprising:
a HCDR1 comprising the amino acid sequence set forth in SEQ ID NO: 1,
a HCDR2 comprising the amino acid sequence set forth in SEQ ID NO: 2, and
a HCDR3 comprising the amino acid sequence set forth in SEQ ID NO: 3; and
a light chain variable region comprising:
a LCDR1 comprising the amino acid sequence set forth in SEQ ID NO: 4,
a LCDR2 comprising the amino acid sequence set forth in SEQ ID NO: 5, and
a LCDR3 comprising the amino acid sequence set forth in SEQ ID NO: 6;
(ii) a heavy chain variable region comprising:
a HCDR1 comprising the amino acid sequence set forth in SEQ ID NO: 9,
a HCDR2 comprising the amino acid sequence set forth in SEQ ID NO: 10, and
a HCDR3 comprising the amino acid sequence set forth in SEQ ID NO: 11; and
a light chain variable region comprising:
a LCDR1 comprising the amino acid sequence set forth in SEQ ID NO: 12,
a LCDR2 comprising the amino acid sequence set forth in SEQ ID NO: 13, and
a LCDR3 comprising the amino acid sequence set forth in SEQ ID NO: 14;
(iii) a heavy chain variable region comprising:
a HCDR1 comprising the amino acid sequence set forth in SEQ ID NO: 17,
a HCDR2 comprising the amino acid sequence set forth in SEQ ID NO: 18, and
a HCDR3 comprising the amino acid sequence set forth in SEQ ID NO: 19; and
a light chain variable region comprising:
a LCDR1 comprising the amino acid sequence set forth in SEQ ID NO: 20,
a LCDR2 comprising the amino acid sequence set forth in SEQ ID NO: 21, and
a LCDR3 comprising the amino acid sequence set forth in SEQ ID NO: 22;
(iv) a heavy chain variable region comprising:
a HCDR1 comprising the amino acid sequence set forth in SEQ ID NO: 25,
a HCDR2 comprising the amino acid sequence set forth in SEQ ID NO: 26, and
a HCDR3 comprising the amino acid sequence set forth in SEQ ID NO: 27; and
a light chain variable region comprising:
a LCDR1 comprising the amino acid sequence set forth in SEQ ID NO: 28,
a LCDR2 comprising the amino acid sequence set forth in SEQ ID NO: 29, and
a LCDR3 comprising the amino acid sequence set forth in SEQ ID NO: 30; or
(v) a heavy chain variable region comprising:
a HCDR1 comprising the amino acid sequence set forth in SEQ ID NO: 33,
a HCDR2 comprising the amino acid sequence set forth in SEQ ID NO: 34, and
a HCDR3 comprising the amino acid sequence set forth in SEQ ID NO: 35; and
a light chain variable region comprising:
a LCDR1 comprising the amino acid sequence set forth in SEQ ID NO: 36,
a LCDR2 comprising the amino acid sequence set forth in SEQ ID NO: 37, and
a LCDR3 comprising the amino acid sequence set forth in SEQ ID NO: 38.

3. An isolated anti-GPRCSD antibody or an antigen-binding fragment thereof, wherein the anti-GPRCSD antibody comprises a heavy chain variable region comprising the HCDR1, the HCDR2, and the HCDR3 of the variable region sequence set forth in SEQ ID NO: 7, 15, 23, 31, or 39, and a light chain variable region comprising the LCDR1, the LCDR2, and the LCDR3 of the variable region sequence set forth in SEQ ID NO: 8, 16, 24, 32, or 40.

4. The isolated anti-GPRCSD antibody or the antigen-binding fragment thereof according to claim 3, wherein the anti-GPRCSD antibody comprises a heavy chain variable region and a light chain variable region; the heavy chain variable region comprises the HCDR1, the HCDR2, and the HCDR3 of the variable region sequence set forth in SEQ ID NO: 7, and the light chain variable region comprises the LCDR1, the LCDR2, and the LCDR3 of the variable region sequence set forth in SEQ ID NO: 8; the heavy chain variable region comprises the HCDR1, the HCDR2, and the HCDR3 of the variable region sequence set forth in SEQ ID NO: 15, and the light chain variable region comprises the LCDR1, the LCDR2, and the LCDR3 of the variable region sequence set forth in SEQ ID NO: 16; the heavy chain variable region comprises the HCDR1, the HCDR2, and the HCDR3 of the variable region sequence set forth in SEQ ID NO: 23, and the light chain variable region comprises the LCDR1, the LCDR2, and the LCDR3 of the variable region sequence set forth in SEQ ID NO: 24; the heavy chain variable region comprises the HCDR1, the HCDR2, and the HCDR3 of the variable region sequence set forth in SEQ ID NO: 31, and the light chain variable region comprises the LCDR1, the LCDR2, and the LCDR3 of the variable region sequence set forth in SEQ ID NO: 32; or the heavy chain variable region comprises the HCDR1, the HCDR2, and the HCDR3 of the variable region sequence set forth in SEQ ID NO: 39, and the light chain variable region comprises the LCDR1, the LCDR2, and the LCDR3 of the variable region sequence set forth in SEQ ID NO: 40.

5. The isolated anti-GPRCSD antibody or the antigen-binding fragment thereof according to any one of claims 1 to 4, wherein the heavy chain variable region comprises an amino acid sequence having at least 80% identity to the sequence set forth in SEQ ID NO: 7, 15, 23, 31, or 39; or/and
the light chain variable region comprises an amino acid sequence having at least 80% identity to the sequence set forth in SEQ ID NO: 8, 16, 24, 32, or 40.

6. The isolated anti-GPRCSD antibody or the antigen-binding fragment thereof according to claim 5, wherein the heavy chain variable region and the light chain variable region are selected from any one of the following:
(i) the heavy chain variable region comprises an amino acid sequence having at least 80% identity to the sequence set forth in SEQ ID NO: 7, and the light chain variable region comprises an amino acid sequence having at least 80% identity to the sequence set forth in SEQ ID NO: 8;
(ii) the heavy chain variable region comprises an amino acid sequence having at least 80% identity to the sequence set forth in SEQ ID NO: 15, and the light chain variable region comprises an amino acid sequence having at least 80% identity to the sequence set forth in SEQ ID NO: 16;
(iii) the heavy chain variable region comprises an amino acid sequence having at least 80% identity to the sequence set forth in SEQ ID NO: 23, and the light chain variable region comprises an amino acid sequence having at least 80% identity to the sequence set forth in SEQ ID NO: 24;
(iv) the heavy chain variable region comprises an amino acid sequence having at least 80% identity to the sequence set forth in SEQ ID NO: 31, and the light chain variable region comprises an amino acid sequence having at least 80% identity to the sequence set forth in SEQ ID NO: 32; or
(v) the heavy chain variable region comprises an amino acid sequence having at least 80% identity to the sequence set forth in SEQ ID NO: 39, and the light chain variable region comprises an amino acid sequence having at least 80% identity to the sequence set forth in SEQ ID NO: 40.

7. The isolated anti-GPRCSD antibody or the antigen-binding fragment thereof according to any one of claims 1 to 6, wherein the antigen-binding fragment is an Fab fragment, an Fab' fragment, an F(ab')₂ fragment, an Fd fragment, an Fv fragment, an isolated CDR region, or an scFv;
optionally, the anti-GPRCSD antibody is a chimeric, humanized, or fully human antibody;
optionally, the anti-GPRCSD antibody is of the IgG1, IgG2, IgG3, or IgG4 isotype.

8. The isolated anti-GPRCSD antibody or the antigen-binding fragment thereof according to any one of claims 1 to 7, wherein the anti-GPRCSD antibody or the antigen-binding fragment thereof binds to a human GPRCSD antigen and optionally, further features any one or more of the following properties:
(1) binding to a monkey GPRCSD antigen;
(2) binding to a mouse GPRCSD antigen; or
(3) no binding to one or more of GPRCSA, GPRCSB, and GPRCSC.

9. The isolated anti-GPRCSD antibody or the antigen-binding fragment thereof according to any one of claims 1 to 8, wherein the anti-GPRCSD antibody is a monospecific antibody or a multispecific antibody.

10. A fusion protein comprising the anti-GPRCSD antibody or the antigen-binding fragment thereof according to any one of claims 1 to 9.

11. A pharmaceutical composition comprising the anti-GPRCSD antibody or the antigen-binding fragment thereof according to any one of claims 1 to 9 or the fusion protein according to claim 10 and further comprising a pharmaceutically acceptable carrier.

12. An isolated nucleic acid encoding the anti-GPRCSD antibody or the antigen-binding fragment thereof according to any one of claims 1 to 9.

13. A vector comprising the nucleic acid according to claim 12.

14. A host cell, comprising the vector according to claim 13 or integrated in the genome with the nucleic acid according to claim 12.

15. A method for preparing the anti-GPRCSD antibody or the antigen-binding fragment thereof according to any one of claims 1 to 9 comprising: culturing a host cell comprising a nucleic acid encoding the GPRCSD antibody or the antigen-binding fragment thereof, and isolating the anti-GPRCSD antibody or the antigen-binding fragment thereof from the host cell or the host cell culture.

16. Use of the anti-GPRCSD antibody or the antigen-binding fragment thereof according to any one of claims 1 to 9 or the fusion protein according to claim 10 for preparing a medicament for treating a disease, reducing a tumor, or inhibiting tumor cell growth, wherein preferably, the disease is a cancer or an autoimmune disease; preferably, the cancer is multiple myeloma.

17. A method for reducing a tumor or inhibiting tumor cell growth in a subject or treating a disease in a subject, comprising administering to the subject a therapeutically effective amount of the anti-GPRCSD antibody or the antigen-binding fragment thereof according to any one of claims 1 to 9, the fusion protein according to claim 10, or the pharmaceutical composition according to claim 11, wherein preferably, the disease is a cancer or an autoimmune disease; preferably, the cancer is multiple myeloma.
